# EUROPEAN PATENT SPECIFICATION

(11) **EP 0 728 147 B1**
(45) Date of publication and mention of the grant of the patent: **12.02.2003**
(21) Application number: 95901112.3
(22) Date of filing: 04.11.1994
(51) Int. Cl.: C07K 14/58, C12N 15/12, A61K 38/22

(54) **RECEPTOR SPECIFIC ATRIAL NATRIURETIC PEPTIDES**
REZEPTOR SPEZIFISCHE ATRIO-NATRIURETISCHE PEPTIDE
PEPTIDES NATRIURETIQUES AURICULAIRES SPECIFIQUES D'UN RECEPTEUR

(30) Priority: 12.11.1993 US 152994
(43) Date of publication of application: 28.08.1996
(73) Proprietor: GENENTECH, INC., South San Francisco, CA 94080-4990 (US)
(72) Inventor: LOWE, David, Brisbane, CA 94005 (US); CUNNINGHAM, Brian, C., Piedmont, CA 94611 (US); OARE, David, Belmont, CA 94002 (US); McDOWELL, Robert, S., San Francisco, CA 94114 (US); BURNIER, John, Pacifica, CA 94044 (US)
(74) Representative: Armitage, Ian Michael
(86) International application number: US9412591
(87) International publication number: WO95013296

(56) References cited:
- EP-A- 0 356 124
- EP-A- 0 465 097
- EMBO JOURNAL, vol. 13, no.11, 1 June 1994 pages 2508-2515, B.C.CUNNINGHAM ET AL 'Production of an ANF variant that is specific for type A receptor'

## Description

### FIELD OF THE INVENTION

The invention relates to synthetic peptides having diuretic, natriuretic, and/or vasodilatory activity in mammals. The peptides of this invention are related to atrial natriuretic peptide (ANP) but exhibit decreased binding affinity to the natriuretic peptide clearance receptor (NPR-C) and improved stability toward peptidases ("enkephalinase") known to hydrolyze natriuretic peptides.

### BACKGROUND OF THE INVENTION

Maintenance of normal extracellular fluid volume depends primarily on the excretion of sodium (natriuresis) and water (diuresis) by the kidneys. These are, in turn, primarily determined by (1) the rate at which plasma is filtered at the glomerulus (glomerular filtration rate, or GFR) and (2) the degree to which sodium is actively reabsorbed along the renal tubule (with water presumably following passively). Sodium reabsorbtion is regulated, in part, by the adrenal steroid hormone aldosterone, in part by blood pressure, hematocrit and plasma viscosity and in part by various atrial natriuretic factors (ANF's) or hormones (deBold, A.J. *et al*., *Life Sciences* **28**:89-94 [1981]; Garcia R, *Experientia* **38**:1071-73 [1982]; Currie, M.S. *et al. Science* **221**:71-73 [1983]; Flynn T.G. *et al*., *Biochem. Biophys. Res. Commun.* **117**:859-865 [1983]; Currie, M.G. *et al*., *Science* **223**:67-69 [1984]; and Kanagawa, K. *et al*., *Biochem. Biophys. Res. Commun.* **118**:131-139 [1984]).

Atrial natriuretic factors are released as a result of sensors in the atrium responsible for detecting extracellular fluid volume. It is believed that an increase in extracellular fluid volume is detected by these sensors as the atrium stretches to accommodate the increased venous return volume. In response to this stimulus ANF is released into the blood stream where it is transported to the kidney. Binding of ANF to a specific natriuretic peptide receptor (hNPR-A) in the kidney causes inhibition of sodium reabsorbtion along the renal tubule decreasing the reabsorption of water and lowering the extracellular fluid volume.

ANF is also known to be a hypotensive hormone that antagonizes various hypertensive systems including; stimulation of vasodialation, inhibition of aldosterone secretion from the adrenal gland, renin secretion from the kidney, and the renin-angiotensin II system.

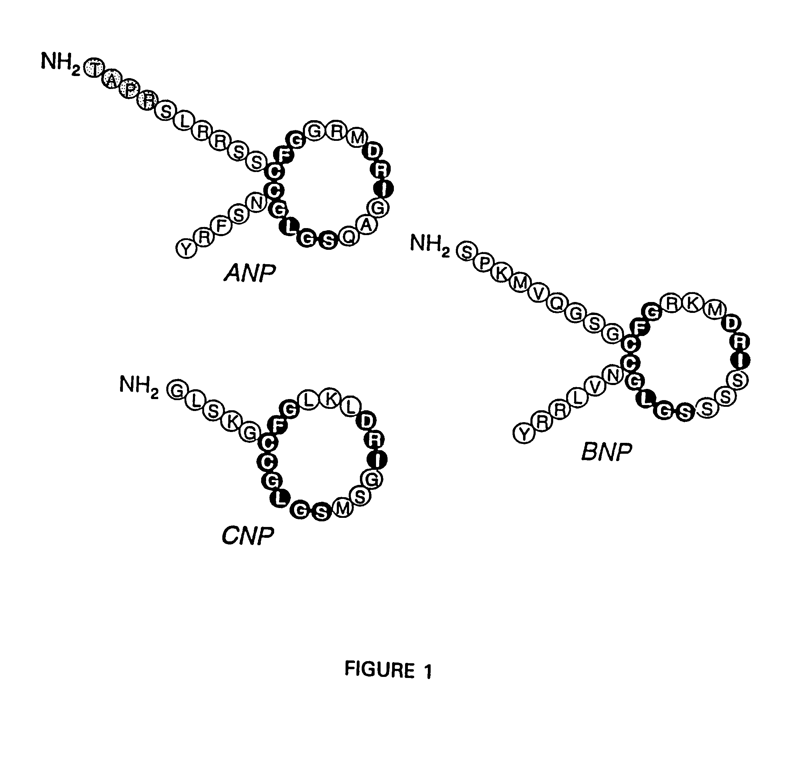

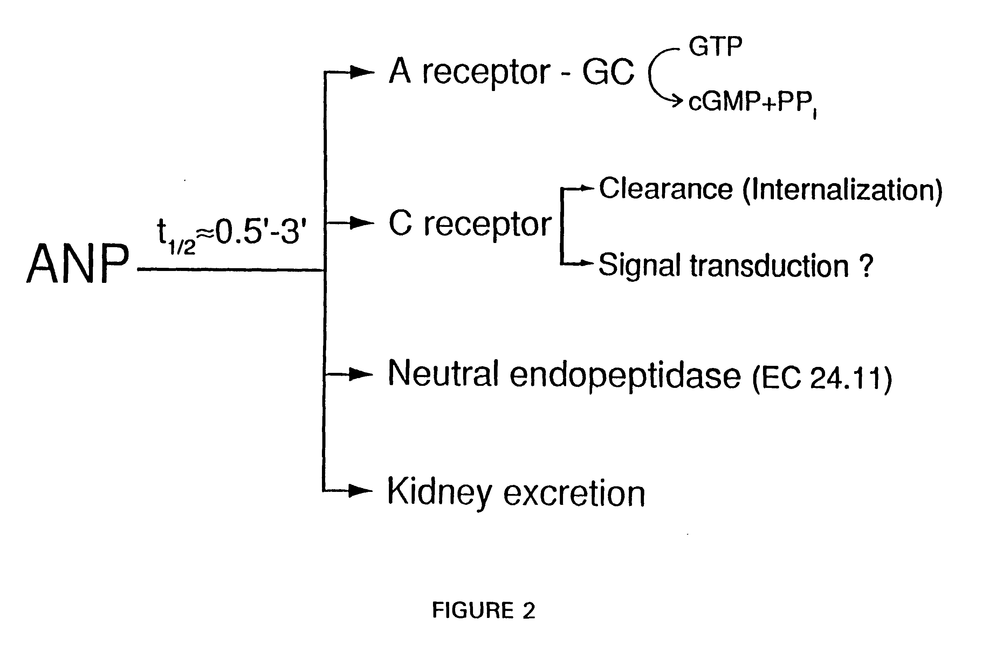

It is known that the serum half-life of ANF's and related peptides (see Fig. 1) that act as hypotensive regulators is relatively short (Crozier, I.G. *et al*., *The Lancet II* 1242-1245 [1986]) and that these peptides are removed from the blood stream by several mechanisms (Fig. 2). In addition to glomerular filtration two other distinct pathways have been identified which appear to significantly contribute to ANF clearance.

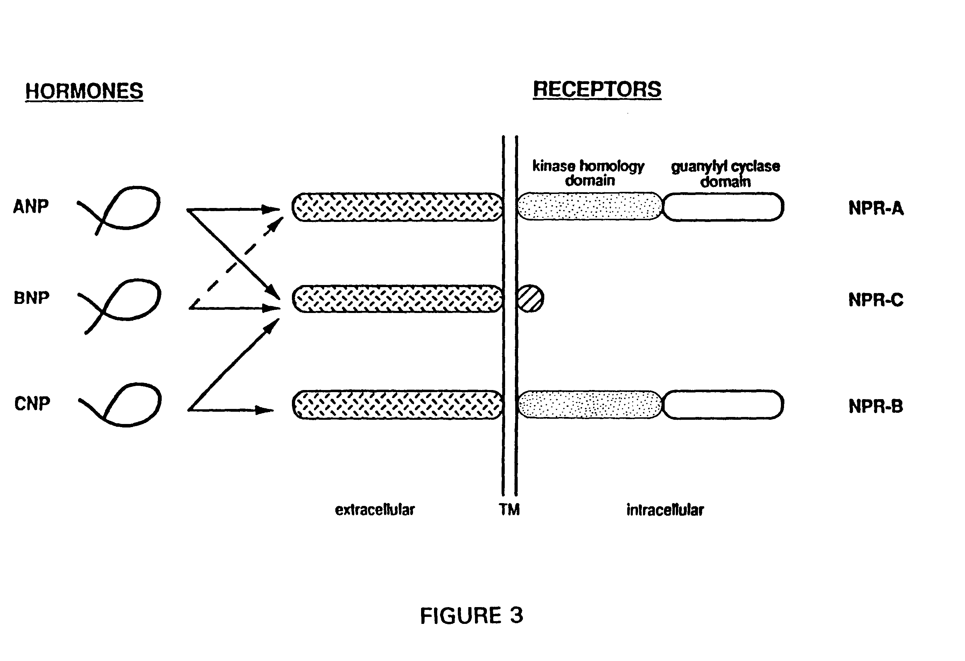

The first of these pathways involves receptor mediated ANF clearance. This pathway is reported to have sufficient capacity to account for about 70-80% of total ANF clearance from the blood stream (Maack, T., *et al, Science* **238**:675-678 [1987], EPO Publication No. 233,143). The human natriuretic peptide receptor (hNPR-C) responsible for this clearance is present in many tissues, especially kidney (Luft, F.C. *et al*., *J. Pharmacol. Exp. Ther.* **236**:416-418 [1986]), and promiscuously (Bovy, P.R., *Med. Res. Rev.* **10**:1156 [1990]) binds various human natriuretic peptides including hANP, hBNP, and hCNP (Fig. 3). Various synthetic peptides, especially linear peptides (Olins, G.M., *et al*., *J. Biol. Chem.* **263**:10989-10993 [1988]), capable of binding the hNPR-C have been described in the patent literature to enhance the natriuretic, diuretic and vasodilatation activity of endogenous ANF. Therapeutic use of these clearance receptor inhibitors presumably elevates the concentration and thus activity of all hormone peptides cleared by hNPR-C.

A second nonsaturatable clearance pathway also operates to remove serum ANF and is believed to involve the activity of a peptidase, neutral endopeptidase 24.11 (EC 3.4.24.11), also known as "enkephalinase" and referred to herein as NEP (Stevenson, S.L., *et al*., *Biochem.* **243**L183-187 [1987]; Olins, G.M., *et al*., *Biochim Biophys Acta* **901**:97-100 [1987]; Koehn, J.A. *et al., J. Biol. Chem.* **262**:11623-11627 [1987]; Yandle, T., *et al*., *Biochem Biophys Res. Commun.* **146**:832-839 [1987]). NEP is present in relatively high amounts in the kidney (Sonnenberg, J.L. *et al*., *Peptides* **9**:173-180 [1988]) and is known to hydrolyze the Cys⁷-Phe⁸ amide bond of ANF's (Tamburine, P.P., *et al*., *J. Pharm. Exp. Ther.* **251**:956-961 [1989]).

It has been observed that inhibitors of NEP, such as thiophan, potentiate the biological responses of administered ANP (Fennell, S.A., *et al*., *FASEB J* **2**:A936 [1988]); Seymour A.A. et al., *ibid;* Trapani, A.J., et al., *ibid;* McMartin, C., et al., *ibid;* Simmerman, M.B. et al. *ibid* A937). However, administration of nonpeptidyl inhibitors of this pathway, like thiorphan, has the disadvantage that the cerebral NEP or endopeptidase 24.11 ("enkephalinase") will also be inhibited because thiorphan is capable of crossing the blood-brain barrier (Bourgoin, S. *et al*., *J. Pharm. Exp. Ther.* **238**:360-366 (1986). In addition to the use of thiorphan, a variety of other strategies for the inhibition of NEP have been described. These strategies include the use of a metal binding substituent appropriately spaced from the aromatic Phe⁸ moiety of ANF. Roques., E.P., *et al*., *Nature* **288**:286-288 (1980); see also Gordon, E.M., *et al*., *Life Sci* **33** (Supplement 1): 113-116 (1983); Mumford, R.M., *et al*., *Biochem Biophys. Res. Comm.* **109**:1303-1309 (1982); Fournie-Zaluski, M.C., *et al*., *J. Med. Chem.* **26**:60-65 (1983); Waksman, G., *et al., Biochem. Biophys. Res. Comm.* **131**:262-268 (1985); U. S. patent no. 5,248,764. Other strategies also include substitution of unnatural residues for Phe⁸ such as cyclohexylamine *(Fed. Proc.,* **45:**657 [1986]; U.S. patent no's. 5,106,834 and 4,861,755) or N-alkylated amino acids like N-Me-Phe (Nutt *et al*., EPA 0 465 097). Introduction of D amino acids such as D-Cys or D-Ala has been described (Nutt R. and Veber, D. F. *Endocrin. Metab, Clin. N. Am.,* **16**:19-41 [1988]; U. S. patent no. 4,816,443) and replacement of amide bonds is described generally be Lewicki *et al*., U. S. patent no. 4,935,492 (see also U. S. patent no. 5,095, 004).

Because of the obvious therapeutic potential of natriuretic peptides and variants thereof in the treatment of congestive heart failure, hypertension, acute kidney failure etc., numerous synthetic ANF's have been prepared that mimic the biological activity of wild-type ANF but are reported to have improved stability, potency, or duration of action when compared to wild-type ANF. Many of these synthetic ANF's are disclosed in the following U.S. Patents: 4,496,544; 4,496,544; 4,609,725; 4,673,732; 4,716,147; 4,757,048; 4,764,504; 4,804,650; 4,816,443; 4,861,755; 4,935,492; 4,952,561; 5,057,495; 5,057,603; 5,091,366; 5,095,004; 5,106,834; 5,159,061; 5,204,328; and 5,212,286. In addition, various foriegn documents describing ANF analogs include: WO85/04870; WO85/04872; WO88/03537; WO88/06596; WO89/10935; WO89/05654; WO90/01940; WO90/14362; WO92/06998; EPA 0 323 740; EPA 0 356 124; EPA 0 291 999; EPA 0 350 318; EPA 0 497 368; EPA 0 385 476; and EPA 0 341 603. None of these publications disclose a hANF variant having human receptor specificity (i.e. high affinity for hNPR-A and low affinity for hNPR-C), nor do they disclose the substitutions to hANF(1-28) residues 9, 11, or 16 necessary to achieve this selectivity. Only WO88/03537 discloses a positively charged residue, D-Arg, at position 16 in a truncated form of ANF.

### SUMMARY OF THE INVENTION

The present invention provides novel compounds as defined by claims 1 and 2. Preferred compounds have potent vasodilatory, natriuretic, and/or hypotensive activity and having enhanced specificity for hNPR-A in relation to other hNPR's, especially hNPR-B and hNPR-C. The compounds of this invention generally have a decreased binding affinity for the human clearance receptor, hNPR-C, compared to the wild-type protein hANF(1-28). These compounds preferably also have an equal or higher affinity for the human A receptor, hNPR-A, compared to the wild-type protein hANF(1-28). These biological characteristics are normally achieved by providing one or more of the following substitutions in wild-type hANF(1-28) or mutants thereof; G9T, G9R, R11S, R11D, G16R, G16K, G16Orn and G16Har. Additionally these compounds have an increased half-life relative to wild-type hANF(1-28 when incubated with neutral endopeptidase 11.24 (NEP). The increased half-life is preferably achieved by; adding the urodilatin amino terminus tetrapeptide sequence to the amino terminus, modifying the Cys⁷-Phe⁸ amide bond with an amide isostere, N-alkylating Cys⁷, Phe⁸ or Ala¹⁷. A preferred compound having hNPR-A selectivity is represented by Formula (I) 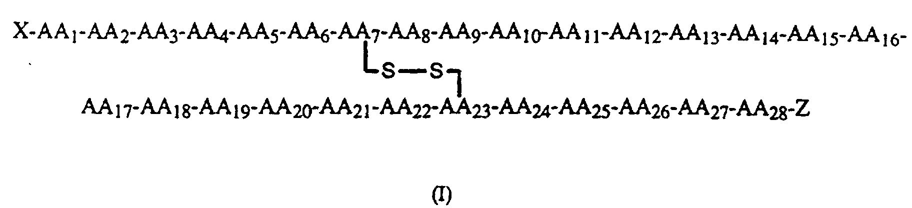 where
X is selected from
   H, C₁-C₆alkanoyl, Ser-Pro-Lys- and Thr-Ala-Pro-Arg- (SEQ ID NO: 1);
AA₁ is absent or is selected from
   Ser, Met, Gly, Asp, Ala, Tyr and His;
AA₂ is absent or is selected from
   Leu, Asp, Met, Val, Ser, Ala, Phe, Pro and Lys;
AA₃ is absent or is selected from
   Cys, Glu, Ser, Gln, His, Gly, Arg and Asp;
AA₄ is absent or is selected from
   Arg, Gly, Ala, Asp, Met, Leu, Tyr and Pro;
AA₅ is absent or is selected from
   Ser, Cys and Asp;
AA₆ is absent or is selected from
   Ser, Gly and Glu;
AA₇ is selected from
   Cys, N-methyl-Cys, D-Cys and Pen;
AA₈ is selected from
   Phe, N-methyl-Phe, Trimethylphenyl-Ala, halo(F, Cl, Br and I)phenyl-Ala, Trifluoromethylphenyl-Ala, Tyr, O-methyl-Tyr, Cha, β-napthyl-Ala, α-napthyl-Ala, biphenyl-Ala, diphenyl-Ala, dibenzyl-Ala, florenyl-Ala, adamantyl-Ala, and β-napthyloxy-Ala;
AA₉ is selected from
   Gly, Arg, Thr, Val, Asp, Ala, Pro and Glu;
AA₁₀ is selected from
   Gly, Arg, Ser, Ala, His, Pro and Lys;
AA₁₁ is selected from
   Arg, Lys, N-methyl-Arg, Asp, Ser and Pro;
AA₁₂ is selected from
   Met, Ile, D-Leu, Nle and Leu;
AA₁₃ is selected from
   Asp and Glu;
AA₁₄ is selected from
   Arg, N-methyl-Arg, Pro and Ser;
AA₁₅ is selected from
   Ile and Leu;
AA₁₆ is selected from
   Gly, Tyr, Phe, Ser, Tyr, Pro and a positively charged amino acid residue selected from
   Orn, Har, Lys, *p*-amidinophenyl-Ala and Arg;
AA₁₇ is selected from
   Ala, Ser, N-methyl-Ala, and Pro;
AA₁₈ is selected from
   Gln, Ser and homo-Cys;
AA₁₉ is selected from
   Ser;
AA₂₀ is selected from
   Gly and Ala;
AA₂₁ is selected from
   Leu;
AA₂₂ is selected from
   Gly and Ala;
AA₂₃ is selected from
   Cys;
AA₂₄ is selected from
   Asn;
AA₂₅ is selected from
   Ser and Val;
AA₂₆ is selected from
   Phe and Leu;
AA₂₇ is selected from
   Arg, Orn, Har, Lys, *p*-amidinophenyl-Ala and Arg-Arg;
AA₂₈ is selected from
   Tyr and homoCys; and
Z is selected from
   OH and NR¹R² where R¹ and R² are independently selected from H, C₁-C₆alkyl, C₆-C₁₂aryl and C₆-C₁₂aryl-C₁-C₆alkyl;
and where
the amide bond (-C(=O)-NH-) bonding residues AA₇ and AA₈ may optionally be replaced with an amide isostere selected from the group
-CH₂-NH-,
-CH₂-S-,
-CH₂-S(O)ₙ-, where n is 1 or 2,
-CH₂-CH₂-,
-CH=CH- (E or Z),
-C(=O)-CH₂-,
-CH(CN)-NH-,
-C(OH)-CH₂-, and
-O-C(=O)-NH-
provided that one of AA₉, AA₁₁, and AA₁₆ is selected according to the following scheme
AA₉ is Arg, Thr or Glu;
AA₁₁ is Asp, Ser or Pro; and
AA₁₆ is a positively charged amino acid residue selected from
Arg, homoArg, Lys, Orn, and *p*-amidinophenyl-Ala; and pharmaceutically acceptable salts thereof, with the proviso that when AA₁₁ is Pro, AA₉ or AA₁₆ are other than Gly, Ala or Pro.

Preferably AA₁₆ in the compound of Formula I is a positively charged amino acid residue selected from; Orn, Har, Lys, *p*-amidinophenyl-Ala or Arg, and most preferably Arg. Also preferably and independently, AA₉ is selected from Arg, Thr, or Glu, and AA₁₁ is selected from Asp, Ser, or Pro. Optionally, AA₃ is Asp, X is Thr-Ala-Pro-Arg- (SEQ ID NO: 1) and/or the compound may contain a second disulfide (or equivalent) bond.

Most preferred hANF variants of this invention include:
hANF(1-28) G9T, R11S, M12I;
hANF(1-28) G9R, R11D, M12I, G16R;
hANF(1-28) G9T, R11S;
hANF(1-28) G9E, G10R, R11P;
hANF(1-28) G10K, R11S;
hANF(1-28) M12I, G16R;
hANF(1-28) G9T, R11S, G16R, Q18P;
hANF(TAPR 1-28) M12I, G16R;
hANF(1-28) M12I, R14S, G16R;
hANF(1-28) G9E, G10R, R11P, M12I, G16R;
hANF(1-28) R3D, G9T, R11S, M12L, R14S, G16R; and
hANF(1-28) R3D, G9T, R11S, M12L, R14S, G16R, Q18 homocysleine, Y28 homocysleine.

The invention further comprises a pharmaceutical composition including a pharmaceutically acceptable excipient and any of the compounds described above. This composition is used in a method for inducing naturesis, diuresis, vasodilation or to modulate the renin-angiotensin II and aldosterone systems.

### BRIEF DESCRIPTION OF THE FIGURES

**FIGURE 1.** Sequence of Wild-Type Human Natriuretic Peptides. Dark circles are conserved residues found in all three human hormones (ANP [SEQ ID NO: 2], BNP [SEQ ID NO: 3] and CNP [SEQ ID NO: 4]). Shaded circles are additional amino-terminus residues found in a kidney-specific human ANF variant named "Urodilatin". Unshaded circles are residues not conserved amoung the human natriuretic hormones.

**FIGURE 2.** The four fates of ANF(or ANP) primairly responsible for its short serum half-life (t_{1/2}).

**FIGURE 3.** Hormone Specificity for Known Natriuretic Peptide Receptors. Solid arrow indicates a strong binding affinity. Dashed arrow indicates a weak binding affinity. The A-receptor (NPR-A) is found predominantly in the adrenal cortex and kidney, the B-receptor (NPR-B) is found predominantly in the atrium of the heart and the central nervous system, while the C-receptor is found primarily in endothelial tissue of the kidney. All three receptors have homologous extracellular domains containing about 440 residues. The C-receptor contains only a small vestigal 37 amino acid intracellular domain of unknown function, while the A- and B- receptors both have guanyl cyclase and tyrosine kinase domains. It is believed the A-receptor is responsible for most of the hornone's hypotensive effects and the C-receptor mediates its rapid clearence.

**FIGURES 4A and 4B.** Resistance to inactivation of ANF variants by cells expressing hNPR-C. In Figure 4A, "ANP" is Wild-type hANF(1-28) while in Figure 4B, the "mutant" is hANF(1-28) R3D, G9T, R11S, M12L, R14S, G16R.

**FIGURE 5.** Binding specificity of mutant hANF(1-28) R3D, G9T, R11S, M12L, R14S, G16R.

**FIGURE 6.** Degradation of ANF variants (0.5 mg/ml) by endopeptidase 11.24 (NEP) (0.225 mg/ml). Peptides of the invention and comparative peptides having the following sequences were assayed as provided in Example 10:

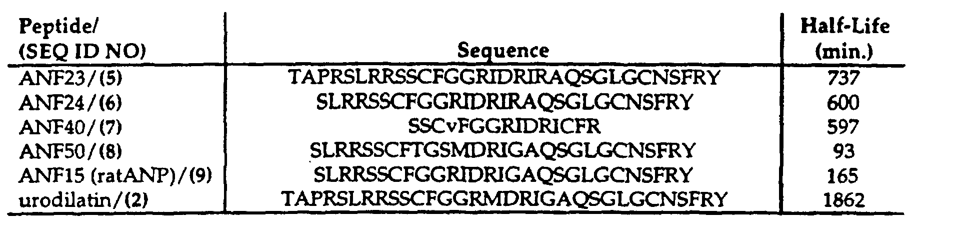

**FIGURE 7.** Degradation of ANF variants (32 nM/ml) by endopeptidase 11.24 (NEP) (0.45 µg/ml). Peptides of the invention and comparative peptides having the following sequences were assayed as provided in Example 10:

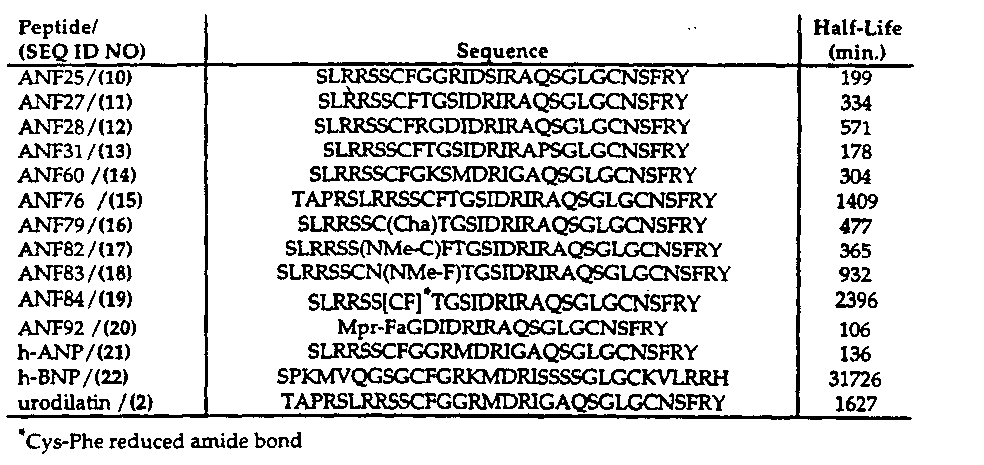

**FIGURE 8.** Degradation of ANF51 (32 nM/ml) by endopeptidase 11.24 (NEP) (0.075 and 0.15 µg/ml). Half-life = 1927 min. @ 0.075 µg/ml NEP and 269 @ 0.15 µg/ml NEP.

### DETAILED DESCRIPTION OF THE INVENTION

### A. Definitions

Terms used in the claims and specification are defined as set forth below unless otherwise specified.

The terms "hANF", "hANF(1-28)", α-hANP and "wild-type hANF" are used interchangeably herein and mean the 28 amino acid residue peptide reported by Kangawa *et al., Biochem. Biophys. Res. Comm.* **118(1)**:131-139 (1984) having the following structure (SEQ ID NO: 21)

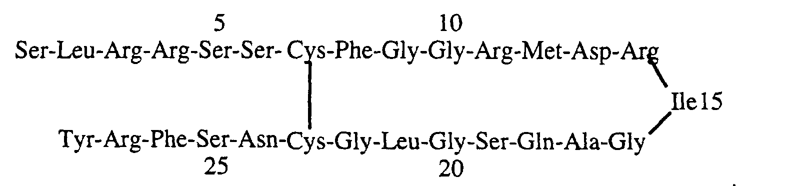

The integers above and below specific residues of this structure define the residue position number. This residue position number is used in conjunction with the one letter amino acid nomenclature, described below, to designate the residue at which a substitution is made in the hANF mutants of this invention. Thus for example, when a mutant hANF is synthesized in which arginine (R) replaces glycine (G) at residue position number 16 of wild-type hANF, the nomenclature "hANF G16R" or "hANF(1-28) G16R" is used. Multiple substitutions are designated in the same manner with a comma separating each substitution as exemplified below.

The term "hANF(1-28) G9T, R11S, G16R" designates a triple mutant hANF having that hANF sequence defined above with the substitution of threonine for glycine at residue position 9 (i.e. G9T), the substitution of serine for arginine at residue position 11 (i.e. R11S), and the substitution of arginine for glycine at position 16 (i.e. G16R). Other mutants are defined in an analogus manner.

The term "hANF(TAPR 1-28) M12I, G16R" designates a double mutant having the tetrapeptide TAPR bonded to the amino group of Ser, residue 1, of hANF.

The term "C₁-C₆alkyl" means a branched, unbranched or cyclic, saturated aliphatic hydrocarbon radical, having the number of carbon atoms specified. Representative examples of these alkyl radicals include methyl, ethyl, n-propyl, isopropyl, n-butyl, iso-butyl, sec-butyl, tert-butyl, n-pentyl, 2-methylbutyl, 2,2-dimethylpropyl, n-hexyl, 2-methylpentyl, 2,2-dimethylbutyl, cycolhexyl and the like. The terms "lower alkyl" and"C₁-C₆alkyl" are synonymous and used interchangeably. A preferred "C₁-C₆alkyl" group is methyl.

The term "C₁-C₆alkanoyl" encompasses groups such as formyl, acetyl, propionyl, butyryl, isobutrryl, valeryl, isovaleryl, pivaloyl, caproyl, and the like.

The term "C₆-C₁₂aryl" means a homocyclic hydrocarbon aromatic radical, whether or not fused, having the number of carbon atoms designated. Preferred aryl groups include phenyl, napthyl, biphenyl, phenanthrenyl, naphthacenyl, and the like (see e.g. *Lang's Handbook of Chemistry* (Dean, J. A., ed) 13^{th} ed. Table 7-2 [1985]).

The term "C₆-C₁₂aryl-C₁-C₆alkyl" means one, two, or three aryl groups having the number of carbon atoms designated, appended to an alkyl radical having the number of carbon atoms designated including but not limited to; benzyl, napthylmethyl, phenethyl, benzyhydryl (diphenylmethyl), florenyl, trityl, and the like. A preferred arylalkyl group is the benzyl group.

Amino acids and amino acid residues described herein may be referred to according to the accepted one or three letter code provided in the table below. Unless otherwise specified these amino acids or residues are of the naturally occurring L stereoisomer form.

| Common Name | One-Letter Symbol | Three-Letter Symbol |
|---|---|---|
| Alanine | A | Ala |
| Arginine | R | Arg |
| Asparagine | N | Asn |
| Aspartic acid | D | Asp |
| Cysteine | C | Cys |
| Glutamine | Q | Gln |
| Glutamic acid | E | Glu |
| Glycine | G | Gly |
| Histidine | H | His |
| Isoleucine | I | Ile |
| Leucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tyrosine | Y | Tyr |
| Valine | V | Val |
| Norleucine | | Nle |
| Cyclohexylalanine | | Cha |
| Homoarginine | A* | Har |
| Ornithine | | Orn |
| Penicillamine | | Pen |
| Phenyl glycine | | Phg |
| Mercaptopropionic acid | | Mpa |
| D Alanine | a | ala |
| Homocysteine | C* | |

In general, unless otherwise specified, the abbreviations used for the designation of amino acids and the protective groups used therefor are based on recommendations of the IUPAC-IUB Commission of Biochemical Nomenclature *(Biochemistry,* **11**:1726-1732 (1972).

The nomenclature used to define compounds of Formula I is that specified by the IUPAC, published in *European Journal of Biochemistry,* **138:**9-37 (1984).

"Pharmaceutically acceptable salts" include both acid and base addition salts.

"Pharmaceutically acceptable acid addition salt" refers to those salts which retain the biological effectiveness and properties of the free bases and which are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, maleic acid, maloneic acid, succinic acid, fumaric acid, tartaric acid, citric acid, benzoic acid, cinnamic acid, mandelic acid, methanesulfonic acid, ethanesulfonic acid, p-toluenesulfonic acid, salicyclic acid and the like.

"Pharmaceutically acceptable base addition salts" include those derived from inorganic bases such as sodium, potassium, lithium, ammonium, calcium, magnesium, iron, zinc, copper, manganese, aluminum salts and the like. Particularly preferred are the ammonium, potassium, sodium, calcium and magnesium salts. Salts derived from pharmaceutically acceptable organic nontoxic bases includes salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperizine, piperidine, N-ethylpiperidine, polyamine resins and the like. Particularly preferred organic non-toxic bases are isopropylamine, diethylamine, ethanolamine, trimethamine, dicyclohexylamine, choline, and caffeine.

### B. Methods of Making

### 1. Chemical Synthesis

### a. General Procedures

One method of producing ANF variants involves chemical synthesis of the polypeptide, followed by treatment under oxidizing conditions appropriate to obtain the native conformation, that is, the correct disulfide bond linkages. This can be accomplished using methodologies well known to those skilled in the art (see Kelley, R.F. & Winkler, M.E. in *Genetic Engineering Principles and Methods,* Setlow, J. K., ed., Plenum Press, N.Y., vol. 12, pp 1-19 [1990]; Stewart, J. M. & Young, J. D. *Solid Phase Peptide Synthesis* Pierce Chemical Co. Rockford, IL[1984]; see also U. S. Patent No.'s 4,105,603; 3,972,859; 3,842,067; and 3,862,925).

Polypeptides of the invention may be conveniently prepared using solid phase peptide synthesis (Merrifield, *J. Am. Chem. Soc.,* **85**:2149 [1964]; Houghten, *Proc. Natl. Acal. Sci. USA* **82:**5132 [1985]). Solid phase synthesis begins at the carboxy-terminus of the putative peptide by coupling a protected amino acid to a suitable resin (e.g. chloromethylated polystyrene resin) as shown in Figures 1-1 and 1-2, on pages 2 and 4 of Stewart and Young *supra.* After removal of the α-amino protecting group with, for example, trifluoroacetic acid (TFA) in methylene chloride and neutralizing in, for example TEA, the next α-amino- and sidechain protected amino acid in the synthesis is added. The remaining α-amino- and, if necessary, side-chain-protected amino acids are then coupled sequentially in the desired order by condensation to obtain an intermediate compound connected to the resin. Alternatively, some amino acids may be coupled to one another forming a peptide prior to addition of the peptide to the growing solid phase polypeptide chain.

The condensation between two amino acids, or an amino acid and a peptide, or a peptide and a peptide can be carried out according to the usual condensation methods such as the azide method, mixed acid anhydride method, DCC (N,N'-dicyclohexylcarbodiimide) or DIC (N,N'-diisopropylcarbodiimide)methods, active ester method (p-nitrophenyl ester method, BOP [benzotriazole-1-yl-oxy-tris (dimethylamino) phosphonium hexafluorophosphate] method, N-hydroxysuccinic acid imido ester method, etc, and Woodward reagent K method.

Common to chemical syntheses of peptides is the protection of any reactive side-chain groups of the amino acids with suitable protecting groups. Ultimately these protecting groups are removed after the desired polypeptide chain has been sequentially assembled. Also common is the protection of the α-amino group on an amino acid or a fragment while that entity reacts at the carboxyl group followed by the selective removal of the α-amino-protecting group to allow subsequent reaction to take place at that location. Accordingly, it is common in polypeptide synthesis that an intermediate compound is produced which contains each of the amino acid residues located in the desired sequence in the peptide chain with various of these residues having side-chain protecting groups attached. These protecting groups are then commonly removed substantially at the same time so as to produce the desired resultant product following removal from the resin.

Suitable protective groups for protecting the α-and ε- amino side chain groups are exemplified by benzyloxycarbonyl (abbreviated Z), isonicotinyloxycarbonyl (iNOC), O-chlorobenzyloxycarbonyl [Z(2Cl] p-nitrobenzyloxycarbonyl [Z(NO₂ ], p-methoxybenzyloxycarbonyl [Z(OMe)], t-butoxycarbonyl, (Boc), t-amyloxycarbonyl (Aoc), isoborrnyloxycarbonyl, adamatyloxycarbonyl, 2-(4-biphenyl)-2-propyloxycarbonyl (Bpoc), 9-fluorenylmethoxycarbonyl (Fmoc), methylsulfonyiethoxycarbonyl (Msc), trifluoroacetyl, phthalyl, formyl, 2-nitrophenylsulphenyl (NPS), diphenylphosphinothioyl (Ppt), dimethylophosphinothioyl (Mpt) and the like.

Protective groups for the carboxy functional group are exemplified by; benzyl ester (OBzl), cyclohexyl ester (Chx), 4-nitrobenzyl ester (ONb), t-butyl ester (Obut), 4-pyridylmethyl ester (OPic), and the like. It is often desirable that specific amino acids such as arginine, cysteine, and serine possessing a functional group other than amino and carboxyl groups are protected by a suitable protective group. For example, the guanidino group of arginine may be protected with nitro, p-toluenesulfonyl, benzyloxycarbonyl, adamantyloxycarbonyl, p-methoxybenzenesulfonyl, 4-methoxy-2, 6-dimethylbenzenesulfonyl (Mds), 1,3,5-trimethylphenysulfonyl (Mts), and the like. The thiol group of cysteine may be protected with p-methoxybenzyl, triphenylmethyl, acetylaminomethyl ethylcarbamoyle, 4-methylbenzyl, 2, 4, 6-trimethy-benzyl (Tmb) etc, and the hydroxyl group of serine can be protected with benzyl, t-butyl, acetyl, tetrahydropyranyl and the like.

Stewart and Young *supra* provides detailed information regarding procedures for preparing peptides. Protection of α-amino groups is described on pages 14-18, and side-chain blockage is described on pages 18-28. A table of protecting groups for amine, hydroxyl and sulfhydryl functions is provided on pages 149-151.

After the desired amino acid sequence has been completed, the intermediate peptide is removed from the resin support by treatment with a reagent, such as liquid HF and one or more thio-containing scavengers, which not only cleaves the peptide from the resin, but also cleaves all the remaining side-chain protecting groups. Following HF cleavage, the protein sequence is washed with ether, transferred to a large volume of dilute acetic acid, and stirred at pH adjusted to about 8.0 with ammonium hydroxide.

Preferably in order to avoid alkylation of residues in the polypeptide, (for example, alkylation of methionine, cysteine, and tyrosine residues) a thio-cresol and cresol scavenger mixture is used. The resin is washed with ether, and immediately transferred to a large volume of dilute acetic acid to solubilize and minimize intermolecular cross-linking. A 250 µM polypeptide concentration is diluted in about 2 liters of 0.1 M acetic acid solution. The solution is then stirred and its pH adjusted to about 8.0 using ammonium hydroxide. Upon pH adjustment, the polypeptide takes its desired conformational arrangement.

### b. Non-peptide (amide isostere) Linkages

In one embodiment of the invention, the amide linkages (-C(=O)-NH-) are replaced with amide isostere linkages such as; -CH₂-NH-, -CH₂-S-, -CH₂-O-, -CH₂-CH₂-, -CH=CH- (cis and trans), -C(=O)-CH₂-, -CH(OH)-CH₂-, -CH(CN)-NH-, -O-C(=O)-NH- and -CH₂-SO-, by methods known in the art. The following references describe preparation of peptide analogs which include these alternative-linking moieties: Spatola, A.F., Vega Data **1**(3): "Peptide Backbone Modifications" (General Review) (Mar 1983), Spatola, A.F., in "Chemistry and biochemistry of Amino Acids Peptides and Proteins", B. Weinstein, ed., Marcel Dekker, New York, P. 267 (1983); Morley, J. S., *Trends Pharm. Sci.* pp. 463-468; Hudson, D. *et al. Int. J. Pept. Prot. Res.* **14**:177-185 (1979) (-CH₂NH-, -CH₂CH₂-); Tourwe, D., *et al*., *Structural Biology* 331-333 (1989) (E, -CH=CH-); Spatola, A.F., *et al*., *Life Sci.* **38**:1243-1249 (1986) (-CH₂-S-); Hann, M.M., *J. Chem. Soc. Perkin. Trans. I* 307-314 (1982) (-CH=CH-, cis and trans); Almquist, R.G., *et al*., *J. Med. Chem.* **23**:1392-1398 (1980) (-C(=O)-CH₂-); Jennings-White C., *et al*., *Tetrahedron Lett* **23**:(1982) (-C(=O)-CH₂-); Szelke, M., *et al.,* EP Application No. 45665 (1982) *Chem Abs* **:97**39405 (1982) (-CH(OH)-CH₂); Holladay, M.W., *et al*., *Tetrahedron Lett* **24**:4401-4404 (1983) (-C(OH)-CH₂-); Hruby, V.J. *Life Sci* **31**:189-199 (1982) (-CH₂S-); and Cho, C.Y. *et al, Science* **261**:1303-1305 (1993) (-O-C(=O)-NH-).

### 2. Recombinant Synthesis

In a further embodiment, the present invention encompasses a composition of matter comprising isolated nucleic acid, preferably DNA, encoding the protein component of a composition of matter comprising a polypeptide selected from ANF variants that contain the amino acid substitutions described in Table I. The invention further comprises an expression control sequence operably linked to the DNA molecule, an expression vector, preferably a plasmid, comprising the DNA molecule, where the control sequence is recognized by a host cell transformed with the vector.

Preferred expression vectors of the present invention may be selected from; pBR322, phGH1, pBO475, pB1537, pRIT5, pRIT2T, pKK233-2, pDR540, pPL-lambda and pB1537, with the most preferred vector being pB1537.

Preferred host cells containing the expression vector of the present invention may be selected from *E. coli* K12 strain 294 (ATCC No. 31446), *E. coli* strain JM101, *E. coli* B, *E. coli* X1776 (ATCC No. 31537), *E. coli* c600, *E. coli* W3110 (F-, gamma-, prototrophic, ATCC No. 27325), *Bacillus subtilis, Salmonella typhimurium, Serratia marcesans,* and *Pseudomonas* species, with the most preferred host cell being *E. coli* W3110 (ATCC No. 27325), or a derivative thereof such as the protease deficient strain 34B8.

The composition of the present invention may be made by a process which includes the steps of isolating or synthesizing (by art standard techniques) nucleic add sequences encoding any of the amino acid sequences described herein, ligating the nucleic acid sequence into a suitable expression vector capable of expressing the nucleic acid sequence in a suitable host, transforming the host with the expression vector into which the nucleic acid sequence has been ligated, culturing the host under conditions suitable for expression of the nucleic acid sequence, whereby the protein encoded by the selected nucleic acid sequence is expressed by the host. In this process, the ligating step may further contemplate ligating the nucleic acid into a suitable expression vector such that the nucleic acid is operably linked to a suitable secretory signal, whereby the amino acid sequence is secreted by the host. The secretory signal may be selected from the group consisting of the leader sequence of stII, ecotin, lamB, herpes gD, lpp, alkaline phosphatase, invertase, and alpha factor and is preferably stII.

### a. Gene Synthesis, Cloning, and Expression

### 1. General Procedures

From the purified protein and its amino acid sequence, ANF or ANF variants may be produced using recombinant DNA techniques. These techniques contemplate, in simplified form, taking a gene encoding either ANF or ANF variants; inserting it into an appropriate vector; inserting the vector into an appropriate host cell; culturing the host cell to cause expression of the ANF or ANF variants gene; and purifying the protein produced thereby.

Somewhat more particularly, the DNA sequence encoding either ANF or ANF variants is cloned and manipulated so that it may be expressed in a convenient host. DNA encoding parent polypeptides can be obtained from a genomic library, from cDNA derived from mRNA from cells expressing ANP or ANP homologs, or by synthetically constructing the DNA sequence (Sambrook, J. *et al,, Molecular Cloning* (2nd ed.), Cold Spring Harbor Laboratory, N.Y. [1989]).

The parent DNA is then inserted into an appropriate plasmid or vector which is used to transform a host cell. In general, plasmid vectors containing replication and control sequences which are derived from species compatible with the host cell are used in connection with those hosts. The vector ordinarily carries a replication site, as well as sequences which encode proteins that are capable of providing phenotypic selection in transformed cells.

For example, *E. coli* may be transformed using pBR322, a plasmid derived from an *E. coli* species (Mandel, M. *et al., J. Mol. Biol.* **53:**154 [1970]). Plasmid pBR322 contains genes for ampicillin and tetracycline resistance, and thus provides easy means for selection. Other vectors include different features such as different promoters, which are often important in expression. For example, plasmids pKK223-3, pDR720, and pPL-lambda represent expression vectors with the tac, trp, or P_{L} promoters that are currently available (Pharmacia Biotechnology).

### 2. Direct expression of ANF or ANF variants

A preferred vector for direct expression is pB1537. This vector was created as described in Example 23-25 and contains origins of replication for *E. coli,* the alkaline phosphatase promoter, the stII signal sequence, the ANP gene, and the ampicillin resistance gene. Other preferred vectors are pR1T5 and pR1T2T (Pharmacia Biotechnology). These vectors contain appropriate promoters followed by the Z domain of protein A, allowing genes inserted into the vectors to be expressed as fusion proteins. Further discussion of these vectors may be found below.

Other preferred vectors can be constructed using standard techniques by combining the relevant traits of the vectors described herein as illustrated in Example 23-25. In this instance a vector containing the origins of replication for phage and *E. coli,* which allow it to be shuttled between such hosts, was used thereby facilitating both mutagenesis and expression (Cunningham, B., *et al*., *Science* **243:**1330-1336 [1989]; Wells, J. & Cunningham, B., WO 90/04788 published 3 May 1990). Relevant traits of the vector include the promoter, the ribosome binding site, the ANF or ANF variant gene or gene fusion (the Z domain of protein A and ANF or ANF variant and its linker), the signal sequence, the antibiotic resistance markers, the copy number, and the appropriate origins of replication.

ANF or ANF variants have been expressed in *E coli* strain 34B8 using a plasmid with the alkaline phosphatase promoter and the stII signal sequence. This resulted in an expression system in which about 12 mg/L of an ANP variant containing the mutations R3D/G9T/R11S/M12L/R14S/G16R and about 3 mg/L of an ANF variant containing just the R3D mutation were secreted into the broth of 10 liter fermentions. Shake flask experiments showed these mutants expressed dramatically higher than wild-type ANF (about 3200 and 800 times greater, respectively). However, by expressing wild-type ANF in a different strain, E coli 23G3, its expression could be increased about 15 fold.

The host cell may be prokaryotic or eukaryotic. Prokaryotes are preferred for cloning and expressing DNA sequences to produce parent polypeptides, segment substituted polypeptides, residue-substituted polypeptides and polypeptide variants. For example, E. *coli* K12 strain 294 (ATCC No. 31446) may be used as *E. coli* B, *E. coli.* X1776 (ATCC No. 31537), and *E. coli* c600 and c600hfl, *E. coli* W3110 (F-, gamma-, prototrophic /ATCC No. 27325), bacilli such as *Bacillus subtilis,* and other enterobacteriaceae such as *Salmonella -typhimurium* or *Serratia marcesans,* and various pseudomonas species. The preferred prokaryote is *E. coli* W3110 (ATCC 27325). When expressed by prokaryotes the polypeptides typically contain an N-terminal methionine or a formyl methionine and are not glycosylated. In the case of fusion proteins, the N-terminal methionine or formyl methionine resides on the amino terminus of the fusion protein or the signal sequence of the fusion protein. These examples are, of course, intended to be illustrative rather than limiting.

In addition to prokaryotes, eukaryotic organisms, such as yeast cultures, or cells derived from multicellular organisms may be used. In principle, any such cell culture is workable. However, interest has been greatest in vertebrate cells, and propagation of vertebrate cells in culture (tissue culture) has become a reproducible procedure (*Tissue Culture,* Academic Press, Kruse and Patterson, editors (1973)). Examples of such useful host cell lines are VERO and HeLa cells, Chinese Hamster Ovary (CHO) cell lines, W138, 293, BHK, COS-7 and MDCK cell lines.

Specifically, in the instant case, the ANF gene was assembled from synthetic oligonucleotides and inserted into a convenient expression vector. The resulting plasmid, pB1537, places the transcription of the ANF gene under the control of the alkaline phosphatase promoter (AP) and translation under control of the tryptophan Shine-Dalgarno sequence. The stII signal sequence was used to secrete ANF into the *E. coli* periplasm.

### 3. Gene Fusions

A variation on the above procedures contemplates the use of gene fusions, wherein the gene encoding ANF or ANF variant is associated, in the vector, with a gene encoding another protein or a fragment of another protein. This results in ANF or ANF variants being produced by the host cell as a fusion with another protein. The "other" protein is often a protein or peptide which can be secreted by the cell, making it possible to isolate and purify the desired protein from the culture medium and eliminating the necessity of destroying the host cells which arises when the desired protein remains inside the cell. Alternatively, the fusion protein can be expressed intracellularly. It is useful to use fusion proteins that are highly expressed.

The use of gene fusions, though not essential, can facilitate the expression of heterologous proteins in *E. coli* as well as the subsequent purification of those gene products (Harris, T. J. R. in *Genetic Engineering,* Williamson, R., Ed., Academic, London, Vol. 4, p. 127[1983]; Uhlen, M. & Moks, T., *Methods Enzymol.* **185:**129-143 [1990]). Protein A fusions are often used because the binding of protein A, or more specifically the Z domain of protein A, to IgG provides an "affinity handle" for the purification of the fused protein (Nilsson, B. & Abrahmsen, L. *Methods Enzymol.* **185**:144-161 [1990]). It has also been shown that many heterologous proteins are degraded when expressed directly in *E. coli,* but are stable when expressed as fusion proteins (Marston, F. A. O., *Biochem J.* **240**: 1 [1986]).

ANF or ANF variants expressed as fusion proteins may be properly folded or may require folding to obtain the native structure. The properly folded fusion protein may be active and useful as pharmaceutical drug. More preferred would be the correctly folded native protein that is obtained from the fusion protein by methods known in the art. Fusion proteins can be cleaved using chemicals, such as cyanogen bromide, which cleaves at a methionine, or hydroxylamine, which cleaves between an Asn and Gly. Using standard recombinant DNA methodology, the nucleotide base pairs encoding these amino acids may be inserted just prior to the 5' end of the ANF or ANF variant gene.

Alternatively, one can employ proteolytic cleavage of fusion proteins, which has been recently reviewed (Carter, P. (1990) in *Protein Purification: From Molecular Mechanisms to Large-Scale Processes,* Ladisch, M. R., Willson, R. C., Painton, C. C., and Builder, S. E., eds., American Chemical Society Symposium Series No. 427, Ch 13, 181-193).

Proteases such Factor Xa, thrombin, subtilisin and mutants thereof, have been successfully used to cleave fusion proteins. Typically, a peptide linker that is amenable to cleavage by the protease used is inserted between the "other" protein (e.g. the Z domain of protein A) and the protein of interest, such as ANF or an ANF variant. Using recombinant DNA methodology, the nucleotide base pairs encoding the linker are inserted between the genes or gene fragments coding for the other proteins. Proteolytic cleavage of the partially purified fusion protein containing the correct linker can then be carried out on either the native fusion protein, or the reduced or denatured fusion protein.

The protein may or may not be properly folded when expressed as a fusion protein. Also, the specific peptide linker containing the cleavage site may or may not be accessible to the protease. These factors determine whether the fusion protein must be denatured and refolded, and if so, whether these procedures are employed before or after cleavage.

When denaturing and refolding are needed, typically the protein is treated with a chaotrope, such a guanidine HCl, and is then treated with a redox buffer, containing, for example, reduced and oxidized dithiothreitol or glutathione at the appropriate ratios, pH, and temperature, such that the protein of interest is refolded to its native structure.

### 4. Mutant DNA Production

As previously discussed, various techniques are also available which may now be employed to produce mutant ANF or an ANF variant DNA, which encodes for additions, deletions, or changes in amino acid sequence of the resultant protein relative to the parent ANF molecule.

By way of illustration, with expression vectors encoding ANF in hand, site specific mutagenesis (Kunkel *et al*., *Methods Enzymol.* 204:125-139 [1991]; Carter, P., *et al*., *Nucl. Acids. Res.* **13**:4331 [1986]; Zoller, M. J. *et al., Nucl. Acids Res.* **10:**6487 [1982]), cassette mutagenesis (Wells, J. A., *et al*., *Gene* **34:**315 [1985]), restriction selection mutagenesis (Wells, J. A., *et al., Philos. Trans, R. Soc. London SerA* **317,** 415 [1986]) or other known techniques may be performed on the ANF DNA. The mutant DNA can then be used in place of the parent DNA by insertion into the aforementioned expression vectors. Growth of host bacteria containing the expression vectors with the mutant DNA allows the production of mutant ANF, which can be isolated as described herein.

Specifically, in the present case, 2 mutants of ANF that substituted aspartic acid at position 3 of ANF for arginine were expressed at dramatically higher levels in *E coli* than wild-type ANF. Additionally, one of these variants that further reduced the basicity of ANF with the mutations R11S and R14S showed the highest level of expression.

### b. Purification and characterization

Purification and characterization of ANF or ANF variants may be carried out by any art standard technique. In the instant case, recombinant mutant ANF (containing the mutations R3D/G9T/R11S/M12L/R14S/G16R) was purified from the broths of *E. coli* cultures grown in 10 liter fermentors by batch reverse phase chromatography, cation exchange chromatography and C18 reverse phase HPLC. Approximately 6 mg of purified ANP mutant per liter of fermentation broth was isolated using this method. The identity of the purified recombinant ANF was verified by mass spectral analysis (3026 +/- 0.4 amu, obs.; 3026 amu, calcd.) and amino acid analysis. The purified ANF appeared to be >95% homogeneous based on reverse phase HPLC. Amino acid analysis of the variants was within experimental error of that calculated from the sequence .

### C. Discovery and Preferred Embodiments

In the course of screening ANF variants in a competition screen for human ANF receptors (hNPR-A vs hNPR-C) a number of unique amino acid substitutions were identified that conferred hNPR-A specificity. Somewhat surprisingly, variants identified in this way, enriched for human NPR-A binding, did not bind rat NPR-A. This species specificity for the variants is consistent with the known sequence difference between rat and human "A" receptors and points out the danger of using receptors other than those of the host in screening for variant ANF's having improved pharmacological properties.

Preferred compounds of this invention are synthetic hormone agonists of the human natriuretic peptide receptor-A (hNPR-A). This receptor is found in kidney, smooth muscle, adrenal and other tissues (Napier M.A. *et al, Proc. Nat. Acad. Sci. USA,* **81**:5946-5950 (1984), and contains an intracellular guanyl cyclase domain. The action of hNPR-A is mediated by hydrolysis of GTP to produce the second messenger cGMP. Accordingly, preferred compounds of this invention stimulate hNPR-A to produce cGMP to at least the same extent as wild-type ANF.

The preferred compounds of this invention do not however bind to the human natriuretic peptide clearance receptor-C (hNPR-C) to the same extent as wild-type ANF. Rather they bind to the hNPR-C with less than 50% and more preferably less than 10% of the affinity of wild-type ANF. This is preferably achieved by substituting residues: T, R and E at position residue 9; S, D and P at position residue 11; and/or R (or another positively charged residue) and sometimes Y at residue position 16 in hANF(1-28).

For assay systems to measure binding of hANF and the compounds of this invention to hNPR-C see: Schank, D. B., *et al*., *Biochem. Biophys. Res. Comm.* **127**:433-442 (1985); Scarborough, R. M., *J. Biol. Chem.* **261**:12960-12964 and WO 90/01940.

The preferred compounds of this invention are also resistant to hydrolysis by NEP or neutral endopeptidase 24.11 (EC3.4.24.11). This is preferably achieved through stabilizing the Cys⁷-Phe⁸ amide bond by replacing it with an amide isostere. Alternatively or additionally, endopeptidase resistance can be improved by adding additional residues to the amino terminus, such as the "urodilatin" TAPR sequence, or by introducing a second crosslinking site into hANF(1-28) or variants thereof. An example of the latter, namely adding a second crosslinking site may be achieved by substituting homoCys into positions 18 and 28 and oxidizing them to form a second disulfide.

In view of the foregoing, most preferred hANF variants include: 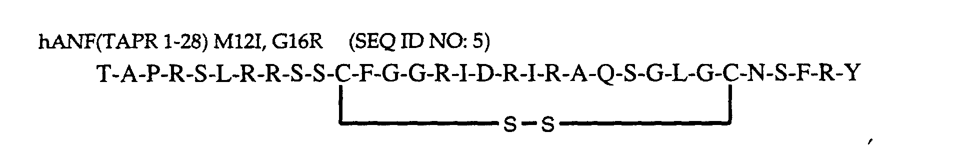 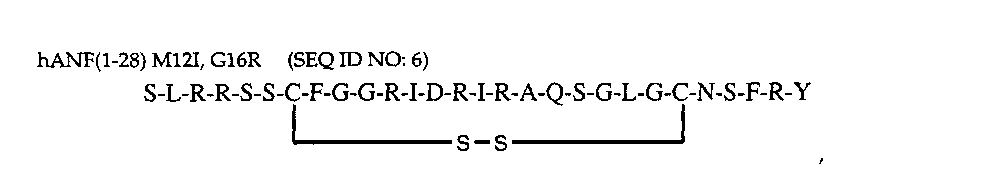 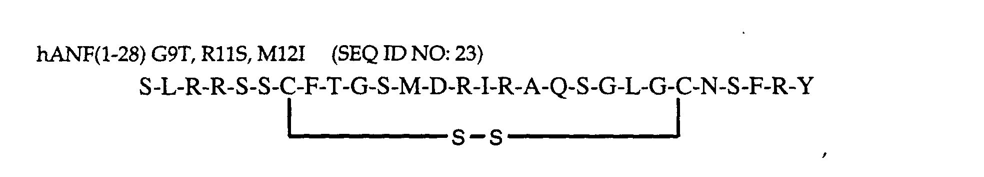 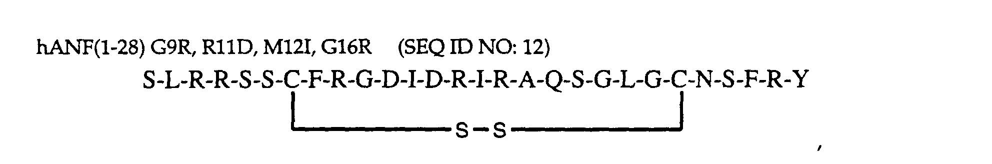 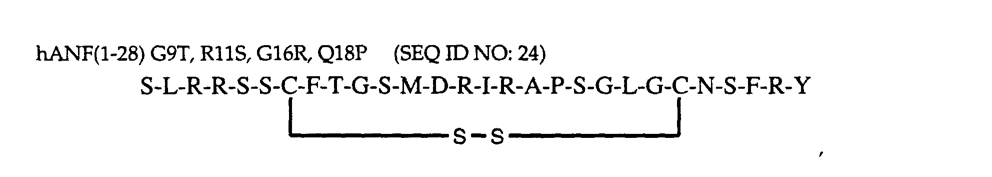 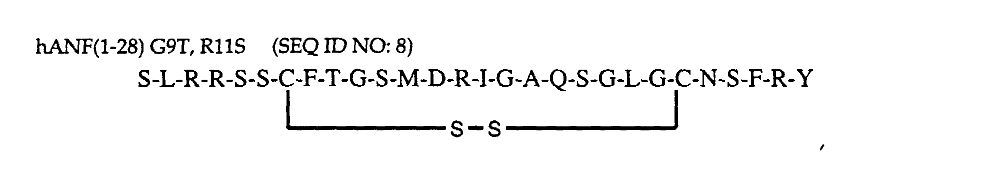 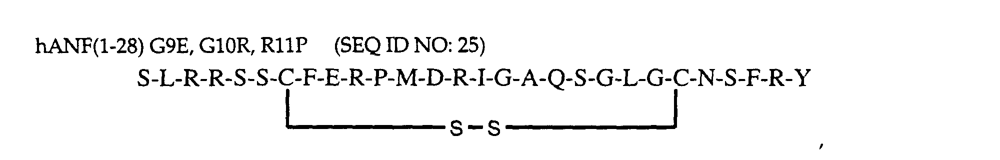 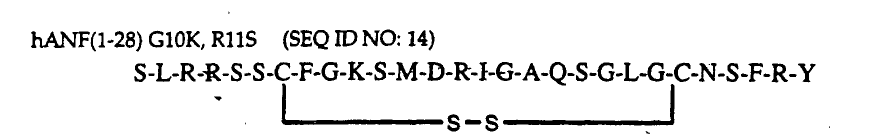 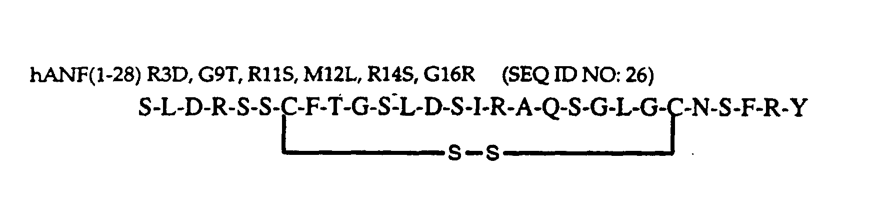 and

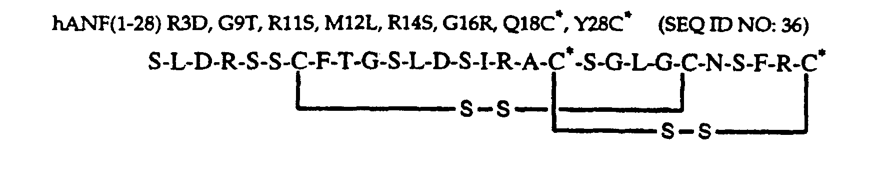

### D. Biological Activity

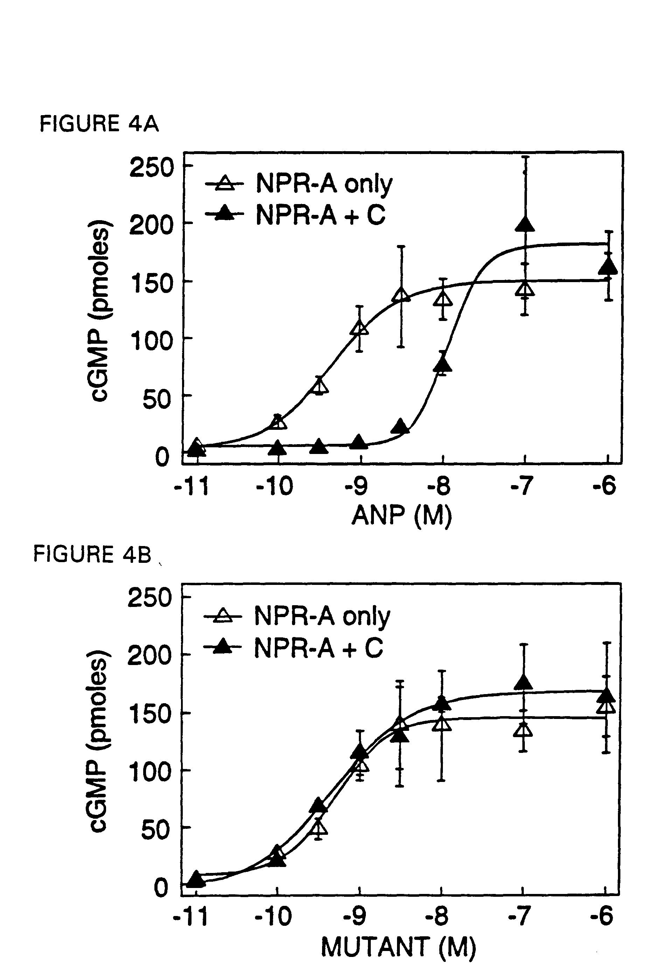

Receptor specific variants of the vasoactive hormone hANF(1-28) have been isolated that stimulate cellular hNPR-A guanyl cyclase activity and block hormone clearance mediated by cultured cells expressing hNPR-C. Figures 4A and 4B demonstrate the resistance to inactivation of a receptor specific ANF variant (hANF(1-28) R3D, G9T, R11S, M12L, R14S, G16R) in a culture of cells expressing hNPR-A alone or in a co-culture of cells expressing both hNPR-A and hNPR-C. Measurement of cGMP production in 293 cells expressing hNPR-A demonstrates that wild-type ANF stimulates guanyl cyclase activity . When a ten-fold excess of 293 cells that highly express hNPR-C (∼10⁶ "C" receptors/cell) is added, the activity of wild-type ANF is greatly reduced (EC₅₀ is about 25-fold higher, see Fig 4A). However, when this experiment is repeated with the receptor specific variant, complete resistance to inactivation is observed (Fig. 4B). Comparison of Fig. 4A and 4B also demonstrates that the receptor specific variant is equipotent to wild-type ANP in stimulating cGMP production from hNPR-A. Based on these findings and the literature reports that hNPR-C mediated clearance accounts for 70-80% of *in vivo* ANF clearance, the variants of this invention are believed to have superior pharmaceutical utility.

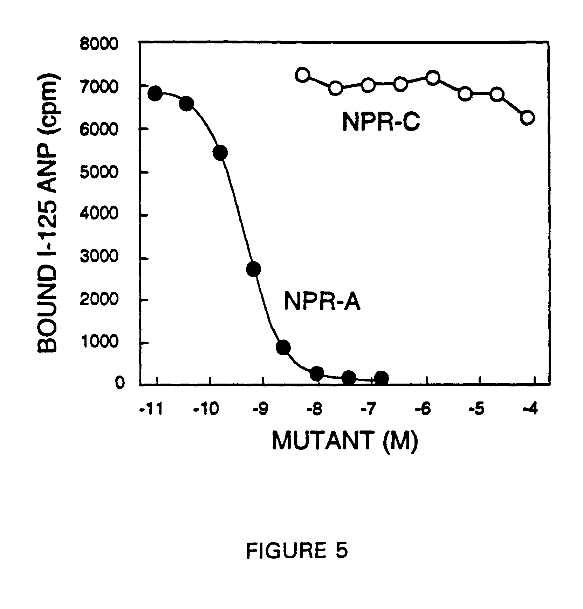

From an analysis of a large number of variants, the single naturally occurring amino acid substitution that produced the greatest receptor specificity was G16R. This substitution had a negligible effect on NPR-A binding but reduced NPR-C affinity about 150-fold. Figure 5 shows the binding specificity for a typical variant containing the G16R substitution. Specifically, variant hANF(1-28) R3D, G9T, R11S, M12L, R14S, G16R competitively displaced bound I¹²⁵ labeled wild-type ANF from hNPR-A but showed no measurable affinity for the clearance receptor hNPR-C. Other receptor specific variants are presented in Table I below.

**TABLE I**

| ANF Variant No. | Structure* (see Formula I) | | | | | | | | Binding Affinity IC₅₀(pM) | |
|---|---|---|---|---|---|---|---|---|---|---|
| | X | AA₉ | AA₁₀ | AA₁₁ | AA₁₂ | AA₁₄ | AA₁₆ | AA₁₈ | hNPR-A | hNPR-C |
| 14 (wild type) | | - | - | - | - | - | - | - | 110 | 230 |
| 23 | TAPR | - | - | - | I | - | R | - | 44 | 4193 |
| 24 | | - | - | - | I | - | R | - | 57 | 5639 |
| 25 | | - | - | - | I | S | R | - | 56 | 3305 |
| 27 | | T | - | S | - | - | R | - | 57 | 1,000,000 |
| 31 | | T | - | S | - | - | R | P | 52 | 3,213,100 |
| 32 | | E | R | P | I | - | R | - | - | - |
| 50 | | T | - | S | - | - | - | - | 570 | 251,000 |
| 51 | | E | R | P | - | - | - | - | 4,550 | 32,200 |
| 60 | | - | K | S | - | - | - | - | 112 | 5980 |
| 70^{a} | | T | - | S | L | S | R | C*-C* | 3,300 | - |

| | | | | | | | | | | |
|---|---|---|---|---|---|---|---|---|---|---|
| * A blank (-) in the table indicates no deviation from the wild type sequence. C*-C* homocys¹⁸-homocys²⁸ disulfide bridges. | | | | | | | | | | |
| ^{a}ANF70 additionally contains a R3D substitution. | | | | | | | | | | |

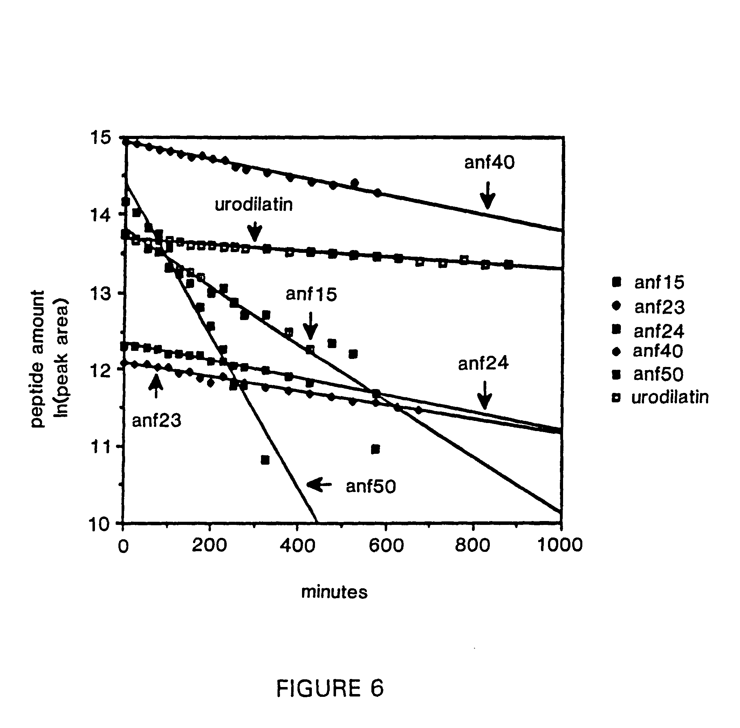

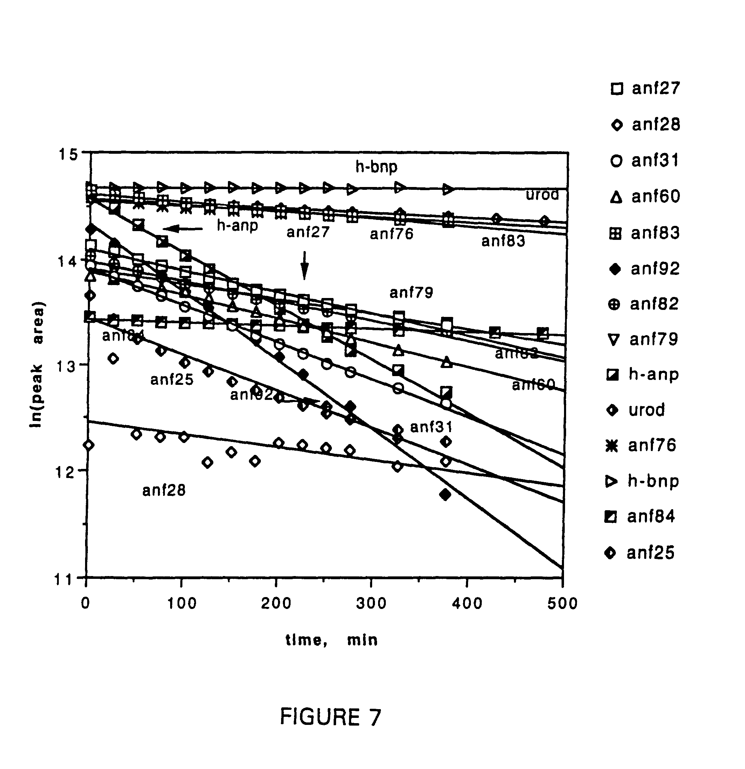

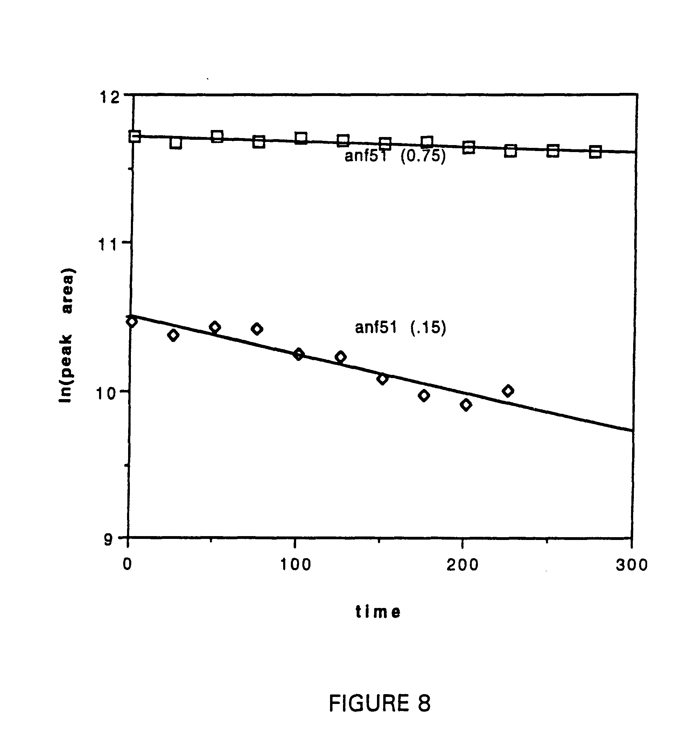

Inhibition of cleavage of ANF variants by human NEP (hNEP) is demonstrated in Figs. 6, 7 and 8. Human Endopeptidase 24.11 inactivates ANF by cleaving the sissile Cys⁷-Phe⁸ amide bond. Assay conditions for measurement of the half-life of various ANF variants is described in Example 10. Half-Lives for variants can be found in the legends (Brief Description of Drawings) to Figs. 6, 7 and 8.

### E. Utility and Administration

Compounds of the present invention have natriuretic, diuretic and vasorelaxant activity and may inhibit the release of aldosterone and renin. Thus, these compounds, find use as therapeutic agents in the treatment of various pathological conditions associated with water or electrolyte imbalance and hypertension, especially renovascular hypertension. Such conditions include, for example, congestive heart failure (CHF), nephrotic syndrome and hepatic cirrhosis, pulmonary disease, and renal failure due to ineffective renal perfusion or reduced glomerular filtration rate.

The present invention also provides compositions containing an effective amount of compounds of the present invention, including the nontoxic addition salts, amides and esters thereof, which may, alone, serve to provide the above-recited therapeutic benefits. Such compositions can be provided together with physiologically tolerable liquid, gel or solid diluents, adjuvants and excipients.

The compounds and compositions can be administered to humans in a manner similar to other therapeutic agents. The dosage to be administered will depend on the usual factors including; age, weight, sex, condition of the patient and route of administration. In general, the dosage required for therapeutic efficacy will range from about 0.01 to 1000 µg/kg, more usually 0.1 to 25 µg/kg of the host body weight. Alternatively, dosages within these ranges can be administered by constant infusion over an extended period of time until the desired therapeutic benefits have been obtained.

Typically, such compositions are prepared as injectable liquid solutions or suspensions. Compositions may also be emulsified. The active ingredient is often mixed with diluents or excipients which are physiologically tolerable and compatible with the active ingredient. Suitable diluents and excipients are, for example, water saline, dextrose, glycerol, or the like, and combinations thereof. In addition, if desired the compositions may contain minor amounts of auxiliary substances such as wetting or emulsifying agents, stabilizing or pH-buffering agents, and the like. For a more detailed description of the foregoing see a standard pharmaceutical text such as *Remington's Pharmaceutical Sciences,* Mack Publishing Co. Easton, PA. (1970).

The compositions of this invention are conventionally administered parenterally by injection, either subcutaneoulsly or intravenously. Additional formulations which are suitable for other modes of administration include suppositories, intranasal aerosols, and, in some cases, oral formulations. For suppositories, traditional binders and excipients may include, for example, polyalkylene glycols or triglycerides; such suppositories may be formed from mixtures containing the active ingredient in the range of 0.5% to 10% preferably 1%-2%. Oral formulations include such normally employed excipients as, for example, pharmaceutical grades of mannitol, lactose, starch, magnesium stearate, sodium saccharin, cellulose, magnesium carbonate, and the like. These compositions take the form of solutions, suspensions, tablets, pills, capsules, sustained-release formulations, or powders, and contain 10%-95% if active ingredient, preferably 25%-70%.

The peptide compounds may be formulated into the compositions as neutral or salt forms. Pharmaceutically acceptable nontoxic salts include the acid addition salts (formed with the free amino groups) and which are formed with inorganic acids such as, for example, hydrochloric or phosphoric acids, or organic acids such as acetic, oxalic, tartaric, mandelic, and the like. Salts formed with the free carboxyl groups may be derived from inorganic bases such as, for example, sodium, potassium, ammonium, calcium, or ferric hydroxides, and such organic bases as isopropyl amine, 2-ethylamino ethanol, histidine, procaine, and the like.

### EXAMPLES

In the following Examples, amino acids are described by the standard three letter amino acid code when referring to intermediates and final products. The linear peptides and intermediates described herein are prepared by the solid phase method of peptide synthesis (R. Merrifield, *J. Am. Chem. Soc.* 1964, **85,** 2149 and M. Bodansky, "Principles of Peptide Synthesis." Springer-Verlag, 1984). Abbreviations used are as follows: tert-butyloxycarbonyl (Boc); p-toluenesulfonyl (Tos); 4-methylbenzyl (MeBzl); benzyl (Bzl); 2-bromobenzyloxycarbonyl (Br-Z); cyclohexyl ester (Ochex); 4-methoxylbenzyl (MeOBzl), 2-chlorobenzyloxycarbonyl (Cl-Z); hydrogen fluoride (HF); benzotriazolyloxytris(dimethylamino)phosphoniumhexafluorophosphate (BOP); methylene chloride (DCM); trifluoroacetic acid (TFA); and dimethylacetamide (DMA).

Peptides were assembled using an Applied Biosystems 430A automated peptide synthesizer. Protected amino acids were obtained from various vendors. Side chain protection was Asp (Ochex), Cys (MeOBzl), Arg (Tos), Ser(Bzl), Thr (Bzl), Lys (Cl-Z), and Tyr (Br-Z).

### Example 1

### Thr-Ala-Pro-Arg-Ser-Leu-Arg-Arg-Ser-Ser-Cys-Phe-Gly-Gly-Arg-Ile-Asp-Arg-Ile-Arg-Ala-Gln-Ser-Gly-Leu-Gly-Cys-Asn-Ser-Phe-Arg-Tyr (SEQ ID NO: 5), Cys⁷-Cys²³ disulfide. (ANF23)

The peptide was assembled on 0.7 g (1.0 mmol) of Boc-Tyr(Br-Z) PAM resin (Applied Biosystems). The coupling protocols were those recommended by Applied Biosystems and DMA was used instead of DMF as solvent. After chain assembly the N-terminal Boc group was removed using TFA. The peptide resin was then washed with dichloromethane followed by methanol and then dried under vacuum. The peptide was then deprotected and removed from the resin by stirring the peptide-resin in 20 ml of a mixture of HF (92.5%), anisole (5%) and ethylmethylsulfide (2.5%) for 1 hr at 0 degrees. The HF was then removed *in vacuo* and the resin washed with ether . The resin was washed with 10% acetic acid in water, acetonitrile, water, and the filtrate was recovered and lyophilized. The crude linear peptide (300 mg) was dissolved in 10 L of distilled water with the pH adjusted to 7.4-7.6 with concentrated ammonium hydroxide. The cyclization is begun immediately by adding 0.003 M K₃[Fe(CN)₆] (1 g dissolved in 100 mL of distilled water) dropwise (ca. 1 drop/10 sec.) with vigorous stirring. Addition of the iron solution is halted when a bright yellow color persists, and the reaction mixture is stirred for an addition 4 to 5 h. The pH of the cyclized solution is adjusted to 4.5 with HOAc.

Bio Rex 70 cation exchange resin , 200-400 mesh in the Na⁺ form is washed with 1 N HCl and then with distilled water. Approximately 110 g of this resin is added to the cyclized peptide solution and stirred overnight. The resin is filtered, washed exhaustively with distilled water, and slurry packed into a column. The peptide is eluted using 70% aqueous HOAc, identified by TLC (ninhydrin visualization) and lyophilized. The peptide was dissolved in 0.1% TFA in water, loaded onto a 2.5 cm x 50 cm C-18 reversed phase column (15 micron, 300A) and eluted with a shallow gradient of increasing acetonitrile. The elution conditions consisted of a 10% to 50% acetonitrile (containing 0.1% TFA) gradient at 0.5% min.⁻¹. The aqueous phase was 0.1% TFA in water. Product containing fractions were then lyophilized to afford the pure title peptide. Electrospray ionization: Electrospray ionization : Calc. M + H = 3586.2; found 3586.3. HPLC t_{R} = 29 min.

Following the procedure in Example 1, the compounds of Examples 2-9 were analogously prepared.

### Example 2

### Ser-Leu-Arg-Arg-Ser-Ser-Cys-Phe-Gly-Gly-Arg-Ile-Asp-Arg-Ile-Arg-Ala-Gln-Ser-Gly-Leu-Gly-Cys-Asn-Ser-Phe-Arg-Tyr (SEQ ID NO: 6), Cys⁷ - Cys²³ disulfide. (ANF24)

Electrospray ionization : Calc. M + H = 3162; found 3161.3. HPLC t_{R} = 31 min.

### Example 3

### Ser-Leu-Arg-Arg-Ser-Ser-Cys-Phe-Thr-Gly-Ser-Ile-Asp-Arg-Ile-Arg-Ala-Gln-Ser-Gly-Leu-Gly-Cys-Asn-Ser-Phe-Arg-Tyr (SEQ ID NO: 11), Cys⁷ - Cys²³ disulfide. (ANF27)

Electrospray ionization : Calc. M + H = **3135.3**; found **3135.6.** HPLC t_{R} = 31 min.

### Example 4

### Ser-Leu-Arg-Arg-Ser-Ser-Cys-Phe-Arg-Gly-Asp-Ile-Asp-Arg-Ile-Arg-Ala-Gln-Ser-Gly-Leu-Gly-Cys-Asn-Ser-Phe-Arg-Tyr (SEQ ID NO: 12), Cys⁷ - Cys²³ disulfide. (ANF 28)

Electrospray ionization : Calc. M + H = 3218.7; found 3219.4. HPLC t_{R} = 30 min.

### Example 5

### Ser-Leu-Arg-Arg-Ser-Ser-Cys-Phe-Thr-Gly-Ser-Ile-Asp-Arg-Ile-Arg-Ala-Pro-Ser-Gly-Leu-Gly-Cys-Asn-Ser-Phe-Arg-Tyr (SEQ ID NO: 13), Cys⁷ - Cys²³ disulfide. (ANF31)

Electrospray ionization : Calc. M + H = 3104.8; found 3104.6. HPLC t_{R} = 30 min.

### Example 6

### Ser-Leu-Arg-Arg-Ser-Ser-Cys-Phe-Thr-Gly-Ser-Met-Asp-Arg-Ile-Gly-Ala-Gln-Ser-Gly-Leu-Gly-Cys-Asn-Ser-Phe-Arg-Tyr (SEQ ID NO: 8), Cys⁷ - Cys²³ disulfide. (ANF50)

Electrospray ionization : Calc. M + H = 3054.4; found 3054.0. HPLC t_{R} = 30 min.

### Example 7

### Ser-Leu-Arg-Arg-Ser-Ser-Cys-Phe-Glu-Arg-Pro-Met-Asp-Arg-Ile-Gly-Ala-Gln-Ser-Gly-Leu-Gly-Cys-Asn-Ser-Phe-Arg-Tyr (SEQ ID NO: 25), Cys⁷ - Cys²³ disulfide. (ANF51)

Electrospray ionization : Calc. M + H = 3190.8; found 3191.9. HPLC t_{R} = 31 min.

### Example 8

### Ser-Leu-Arg-Arg-Ser-Ser-Cys-Phe-Gly-Lys-Ser-Met-Asp-Arg-Ile-Gly-Ala-Gln-Ser-Gly-Leu-Gly-Cys-Asn-Ser-Phe-Arg-Tyr (SEQ ID NO: 14), Cys⁷ - Cys²³ disulfide. (ANF60)

Electrospray ionization : Calc. M + H = 3082.4; found 3082.3. HPLC t_{R} = 28 min..

### Example 9

### Ser-Leu-Asp-Arg-Ser-Ser-Cys-Phe-Thr-Gly-Ser-Leu-Asp-Ser-Ile-Arg-Ala-homocysteine-Ser-Gly-Leu-Gly-Cys-Asn-Ser-Phe-Arg-homocysteine (SEQ ID NO: 37) Cys⁷ - Cys²³ disulfide, homocysteine¹⁸-homocysteine²⁸ disulfide. (ANF70)

The following standard manual peptide synthesis protocol was employed in the construction of this multiple disulfied variant ("tie-down" variant):
(1) Wash 3x with dichloromethane (DCM)
(2) Treat for 1 min. with 50:50 TFA/DCM
(3) Treat for 20 min. with 50:50 TFA/DCM
(4) Wash 3 x with DCM
(5) Treat for 1 min. with 5 % (v/v) DIPEA/DCM.
(6) Treat for 5 min. with 5 % (v/v) DIPEA/DCM.
(7) Wash 4 x with DCM.
(8) Wash 1 x with N-methyl-2-pyrroldinone (NMP).
(9) Add 5 equivalents of the Boc-protected amino acid 5 equivalents of diisopropylcarbodiimide (DIPC) and 5 equivalents of hydroxybenzotriazole (HOBT) in 50:50 DCM/NMP.
(10) Wash 1 x with NMP.
(11) Wash 3 x with DCM.
(12) Test by ninhydrin reaction according to Kaiser et al., *Anal. Biochem.* 1970, 34, 595. If the coupling reaction is incomplete, repeat steps 5-12.

The peptide was synthesized manually, starting with 1.5 g (0.92 mmol) of Boc-homocysteine (MeBzl) Merrified resin. The peptide was synthesized using standard t-Boc chemistry protocol. Cysteine⁷ and cysteine²³ side chains were protected as acetamidomethyl (Acm) thioethers while homocysteine¹⁸ and homocysteine²⁸ side chain were protected as methyl benzyl (MeBzl) thioethers. The side chain carboxylate of Asp¹³ was protected as the benzyl ester; the carboxylate side chain of Asp³ was protected as a cyclohexyl ester. After peptide chain assembly, the N-terminal Boc group was removed using TFA. The peptide resin was then washed with dichloromethane followed by ethanol and dried under vacuum. The peptide was then deprotected and removed from the resin by stirring the peptide resin in 40 mL of a mixture of HF (92.5%), anisole (5%) and ethyl methyl sulfide (2.5%) for one hour in an ice bath. The HF was then removed in vacuo and resin was washed with ether. The peptide was extracted from resin by washing with 10% acetic acid in water followed by water. The filtrate was pooled and lyophilized. The crude linear peptide (400 mg) was dissolved in 800 ml of water with pH adjusted to 8.0 with ammonium hydroxide. The solution was stirred for 72 hours at room temperature after which solution was acidified with TFA, filtered and loaded onto a 1.0 cm x 50 cm C-18 reversed phase column (15 micron, 300 A). The peptide was eluted with a shallow gradient of 10% to 50% acetonitrile (containing 0.1% TFA) with increasing acetonitrile at 0.5%/min. The aqueous phase was 0.1% TFA in water. The intermediate product (homocys¹⁸, homocys²⁸ disulfide) fractious were lyophilized. Electron spray ionization: calc. M + H = 3110.3, found = 3110.0. HPLC tR = 38 min. The intermediate product (homocys¹⁸, homocys²⁸ disulfide - 20 mg) was dissolved in 40 mLof acetic acid: water (80:20). A solution of iodine was prepared in glacial acetic acid and added dropwise to peptide containing solution over a period of time until brownish yellow color persisted. The mixture was stirred overnight (20 hr). Small amounts of zinc dust were added to the mixture the next day until solution became colorless. The solution was filtered, diluted with water and lyophilized. The lyophilized crude peptide was dissolved in 50 ml of 5% acetonitrile in water, filtered and loaded onto a 1 x 50 cm Vydac (18 column - 15 - 20 micron, 300 A) for HPLC purification.

The peptide was eluted with a shallow gradient of 17% to 32% acetonitrile (containing 0.1% TFA) with increasing acetonitrile at 0.25% min.⁻¹. The product containing fractions were lyophilized: Electrospray ionization : Calc. M + H = 2966.2; found 2966.69.

### Example 10

### Inhibition of cleavage of ANF variants by human NEP (hNEP)

Human endopeptidase 24.11 inactivates ANF by cleaving the sissile Cys⁷-Phe⁸ amide bond. The half-life of various ANF variants was measured according to the following procedure.

*ANF Solution:* An accurately weighed amount of ANF or variant thereof was diluted with HEPES buffer (5 ml of 1 M HEPES Buffer and 0.1 ml of Tween 20 diluted to a 100 ml volume with DI water) to make a 0.5 mg/ml solution.

*hNEP*/*HEPES Solution:* 7.5 µl of a 1 mg/ml solution of hNEP (see U. S. patent no. 4,960,700) is dissolved in 992.5 µl of HEPES buffer (as described above) and vortex mixed. The resulting mixture is stored in a silylated Eppendorf tube and periodically checked for activity against an ANF peptide standard.

*Assay conditions:* 150 µl of an ANF solution in the HEPES buffer is diluted with 820 µl HEPES buffer and vortex mixed (1 sec). Careful note of the time is taken as 30 µl of the hNEP/HEPES solution is added and the solution is vortex mixed for 1 sec. and analyzed by periodic removal of constant volume aliquots by the HPLC autosampler. A 2.5 cm x 50 cm, C-18 reversed-phase column (15 micron, 300 Å]) was used and elution was achieved by using a shallow gradient of increasing acetonitrile. The elution conditions consisted of a 10% to 50% acetonitrile (containing 0.1% TFA) gradient at 0.5% min.⁻¹. The aqueous phase was 0.1% TFA in water. The peak area of the ANP peptide is recorded at these periodic intervals and a half-life is determined from these values.

In the recombinant methods described below in Examples 10-13 the following materials and methods were used. The Nugel P-RP reverse phase resin was purchased from Separation Industries and the Vydac C18 and Mono S columns from Pharmacia LKB Biotechnology, Inc. Oligonucleotides were synthesized using hydrogen phosphonate chemistry (Froehler, B. C. *et al*., *Nucleic Acids Res.* **14**:5399-5407 [1986]) and purified by polyacrylamide gel electrophoresis (PAGE). *E. coli* strain XL1-Blue MRF' was purchased from Stratagene. Restriction enzymes were from New England Biolabs. All other reagents were obtained were of the highest grade commercially available.

### Example 11

### Construction of the ANF Expression Vector

All DNA manipulations were performed according to standard procedures unless otherwise indicated (Sambrook *et al*., *supra*). The ANF gene was assembled from synthetic oligonucleotides designed to code for the published sequence of the ANF and code for the restriction sites Nsi I and Apa at the 5' and 3' ends of the gene, respectively. Three sense strand oligonucleotides and 3 overlapping anti-sense strand oligonucleotides were synthesized. The sequence of the sense strands were; The sequence of the anti-sense strands were; These oligonucleotides were phosphorylated, ligated together and the ligation product digested with Nsi I and Apa I after heat inactivation of the ligase. The digest was then electrophoresed on a 5% polyacrylamide gel in 89 mM Tris-borate, 1 mM EDTA, pH 7.9, the gel was stained with ethidium bromide, and a band of approximately 95 base pairs was excised and the DNA electro-eluted. The ANF expression vector pB1325 was created by ligating the fragment obtained from the oligonucleotide assembly into the large vector fragment of Nsi I and Apa I digested pS0132. After ligation *E coli* XL-1 cells were transformed with the mixture and ampicillin resistent colonies were selected. DNA was subsequently isolated from a number of these colonies and subjected to dideoxy sequencing (Sanger, F., Nicklen, S., and A. R. Coulsen, *Proc. Natl, Acad. Sci. USA* 74:5463-5467 [1977]). A plasmid which encoded the correct ANP sequence, pB1325, was isolated and further subjected to site-directed mutagenesis (Kunkel) using an oligonucleotide with the sequence; 5'-AACAGCTTCCGTTACTAGGGCGGGCCC (SEQ ID NO: 44) to introduce an amber stop codon immediately following the codon for Tyr28 of the ANF sequence. The resulting plasmid, pB1537, contains the wild-type human ANF gene under the expression control of the alkaline phosphatase promoter, the trytophan Shine-Dalgarno sequence and the stII signal sequence. Shake flask experiments showed that pB1537 in 23G3 would secrete small amounts of ANF into the culture broth. However, some ANF variants in the same expression vector were found to express much higher amounts of protein.

### Example 12

### Expression and Purification of Recombinant ANF Variant ANF(1-28) R3D, G9T, R11S, M12L, R14S, G16R

Cultures of 34B8 pB1761T (see Example 11 for DNA construction) were grown at 37°C for about 24 hours in an aerated 10 liter fermentor in a low phosphate minimal media (Carter, P. et al., Biotechnology 10 pp163-167, 1992) and containing 2 mg/ml carbenicillin. Glucose was added to maintain glucose excess or avoid anaerobiosis, depending on cell density, and the pH was maintained at pH 7.4 with the addition of NH₄OH. Cell density at harvest was about 100 OD₅₅₀.

Recombinant ANF containing the mutations R3D/G9T/R11S/M12L/R14S/G16R was purified as follows. The above cells were removed from 1 liter of fermentation culture by centrifugation at 6000 rpm (in Sorvall GS3 rotor) for 15 min, and the broth further clarified by filtration through a 0.45 micron membrane. The broth was brought to 0.1 % TCA, incubated at room temperature for 30 minutes and centrifuged at 9000 rpm (in Sorvall GS3 rotor) for 30 minutes to remove any precipitable material. To the supernatant 1/10 volume of Nugel P-RP (Separation Industries) was added and gently mixed for 1 hour at room temperature. The reverse phase resin was then separated with a sintered glass filter and rinsed extensively with 0.1 % TFA. The bound material was then eluted with 50 % acetonitrile + 0.1 % TFA. The elute was then concentrated with a rotovap to remove the acetonitrile, and resuspended in 50 ml of 10mM acetate buffer, pH 4.6. This solution was loaded onto a Mono S column, thoroughly rinsed with 20mM acetate buffer, pH 4.6, and eluted with a 0-200 mM NaCl gradient in 20 mM acetate buffer, pH 4.6. The eluted fractions assayed to contain ANP activity were then pooled, concentrated by lyophilization, and resuspended in 5 ml of dH₂O. This sample was acidified (final 0.1 % TFA), loaded onto a preparative Vydac C18 column and fractioned over a 0-30 % acetonitrile + 0.1% TFA gradient. A large dominant peak that occurred at about 16 % acetonitrile was collected. Mass specroscopy, amino acid analysis and NMR showed this to the expected mutant ANP at a purity of greater than 95%. This material was lyophilized for storage.

### Example 13

### ANF Variants

The ANF expression vector, pB1537, contains a fl origin of replication that enables the production of single stranded DNA which can be used for site-directed mutagenesis of this plasmid. Oligonucleotides with the sequences
5'-GCCTATGCATCTCTGGATAGATCTAGCTGC (SEQ ID NO: 45) and
5'-AGATCTAGCTGCTTCACCGGCAGCCTGGATAGTATCAGAGCTCAGAGCGG (SEQ ID NO: 46) were used to separately introduce the ANF mutations R3D and G9T/R11S/M12L/R14S/G16R into pB1537 by site-directed mutagenesis as described by Kunkel (Kunkel, T. A. *Proc. Natl. Acad. Sci USA* 82:488-492 [1985]). To produce a mutant combining the mutations generated from both oligonucleotides the ∼900 bp Bgl II-Sty1 DNA fragment from the R3D mutant was ligated to the ∼4000 bp Bgl II-Sty1 DNA fragment of the G9T/R11S/M12L/R14S/G16R mutant. The resulting plasmid codes for the ANF mutant R3D/G9T/R11S/M12L/R14S/G16R is referred to as pB1761T.

## Claims

1. A compound represented by Formula (I) 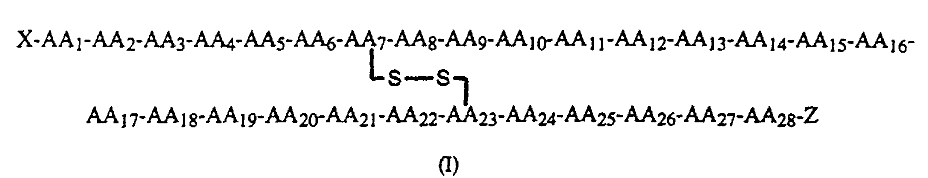 where
X is selected from
H, C₁-C₆alkanoyl, Ser-Pro-Lys-, Thr-Ala-Pro-Arg- (SEQ ID NO: 1) and C₁-C₆alkanoyl-Thr-Ala-Pro-Arg- (SEQ ID NO: 1);
AA₁ is absent or is selected from
Ser, Met, Gly, Asp, Ala, Tyr and His;
AA₂ is absent or is selected from
Leu, Asp, Met, Val, Ser, Ala, Phe, Pro and Lys;
AA₃ is absent or is selected from
Cys, Glu, Ser, Gin, His, Gly, Arg and Asp;
AA₄ is absent or is selected from
Arg, Gly, Ala, Asp, Met, Leu, Tyr and Pro;
AA₅ is absent or is selected from
Ser, Cys and Asp;
AA₆ is absent or is selected from
Ser, Gly and Glu;
AA₇ is selected from
Cys, N-methyl-Cys, D-Cys and Pen;
AA₈ is selected from
Phe, N-methyl-Phe, Trimethylphenyl-Ala, halo(F, Cl, Br and I)phenyl-Ala, Trifluoromethylphenyl-Ala, Tyr, O-methyl-Tyr, Cha, β-napthyl-Ala, α-napthyl-Ala, biphenyl-Ala, diphenyl-Ala, dibenzyl-Ala, florenyl-Ala, adamantyl-Ala, and β-napthyloxy-Ala;
AA₉ is selected from
Gly, Arg, Thr, Val, Asp, Ala, Pro and Glu;
AA₁₀ is selected from
Gly, Arg, Ser, Ala, His, Pro and Lys;
AA₁₁ is selected from
Arg, Lys, N-methyl-Arg, Asp, Ser and Pro;
AA₁₂ is selected from
Met, Ile, D-Leu, Nle and Leu;
AA₁₃ is selected from
Asp and Glu;
AA₁₄ is selected from
Arg, N-methyl-Arg, Pro and Ser;
AA₁₅ is selected from
Ile and Leu;
AA₁₆ is selected from
Gly, Tyr, Phe, Ser, Tyr, Pro and a positively charged amino acid residue selected from Orn, Har, Lys, *p*-amidinophenyl-Ala and Arg;
AA₁₇ is selected from
Ala, Ser, N-methyl-Ala, and Pro;
AA₁₈ is selected from
Gln, Ser and homo-Cys;
AA₁₉ is selected from
Ser;
AA₂₀ is selected from
Gly and Ala;
AA₂₁ is selected from
Leu;
AA₂₂ is selected from
Gly and Ala;
AA₂₃ is selected from
Cys;
AA₂₄ is selected from
Asn;
AA₂₅ is selected from
Ser and Val;
AA₂₆ is selected from
Phe and Leu;
AA₂₇ is selected from
Arg, Orn, Har, Lys, *p*-amidinophenyl-Ala and Arg-Arg;
AA₂₈ is selected from
Tyr, Arg, Orn, Har, Lys, *p*-amidinophenyl-Ala, and homoCys; and
Z is selected from
OH and NR¹R² where R¹ and R² are independently selected from H, C₁-C₆alkyl, C₆-C₁₂aryl and C₆-C₁₂aryl-C₁-C₆alkyl;
and where
the amide bond (-C(=O)-NH-) bonding residues AA₇ and AA₈ may optionally be replaced with an amide isostere selected from the group
-CH₂-NH-,
-CH₂-S-,
-CH₂-S(O)ₙ-, where n is 1 or 2,
-CH₂-CH₂-,
-CH=CH- (E or Z),
-C(=O)-CH₂-,
-CH(CN)-NH-,
-C(OH)-CH₂-, and
-O-C(=O)-NH-
provided that one of AA₉, AA₁₁, and AA₁₆ is selected according to the following scheme
AA₉ is Arg, Thr or Glu;
AA₁₁ is Asp, Ser or Pro; and
AA₁₆ is a positively charged amino acid residue selected from
Arg, homoArg, Lys, Orn, and *p*-amidinophenyl-Ala; and pharmaceutically acceptable salts thereof, with the proviso that when AA₁₁ is Pro, AA₉ or AA₁₆ are other than Gly, Ala or Pro.

2. A compound represented by Formula (II) (SEQ ID NO: 47) 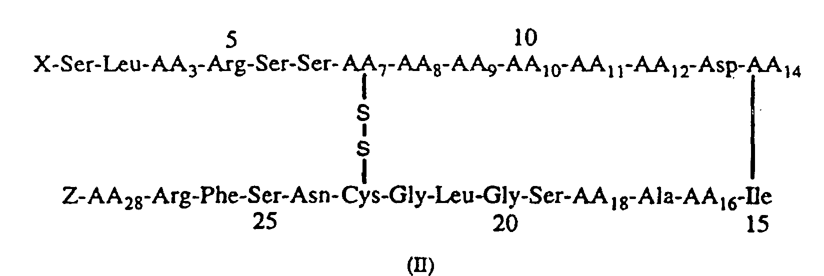 where
X is selected from
H, C₁-C₆alkanoyl, and Thr-Ala-Pro-Arg- (SEQ ID NO: 1);
AA₃ is selected from
Arg or Asp;
AA₇ is selected from
Cys, N-methyl-Cys, D-Cys and Pen;
AA₈ is selected from
Phe, N-methyl-Phe, trimethylphenyl-Ala, flourophenyl-Ala, trifluoromethylphenyl-Ala, Tyr, O-methyl-Tyr, Cha, β-napthyl-Ala, α-napthyl-Ala, biphenyl-Ala, diphenyl-Ala, dibenzyl-Ala, florenyl-Ala, adamantyl-Ala, 2-thienyl-Ala and β-napthyloxy-Ala;
AA₉ is selected from
Gly, Arg, Thr, Val, Asp and Glu;
AA₁₀ is selected from
Gly, Arg, Ser, Ala, His and Lys;
AA₁₁ is selected from
Arg, Asp, Ser and Pro;
AA₁₂ is selected from
Met, Ile, D-Leu, Leu and Nle;
AA₁₄ is selected from
Arg and Ser;
AA₁₆ is selected from
Gly, Phe, Tyr, and a positively charged amino acid residue selected from
Orn, Har, Lys, *p*-amidinophenyl-Ala and Arg;
AA₁₈ is selected from
Gln and homoCys;
AA₂₈ is selected from
Tyr and homoCys; and
Z is selected from
OH and NH₂;
and where
the amide bond (-C(=O)-NH-) bonding residues AA₇ and AA₈ may optionally be replaced with an amide isostere selected from the group
-CH₂-NH-,
-CH₂-S-,
-CH₂-S(O)ₙ-, where n is 1 or 2,
-CH₂-CH₂-,
-CH=CH- (E or Z),
-C(=O)-CH₂-,
-CH(CN)-NH-,
-C(OH)-CH₂-, and
-O-C(=O)-NH-
provided that one of AA₉, AA₁₁, and AA₁₆ is selected according to the following scheme
AA₉ is Arg, Thr or Glu;
AA₁₁ is Asp, Ser or Pro; and
AA₁₆ is a positively charged amino acid residue selected from
Om, Har, Lys, *p*-amidinophenyl-Ala and Arg; and
pharmaceutically acceptable salts thereof, with the proviso that when AA₁₁ is Pro, AA₉ or AA₁₆ are other than Gly, Ala or Pro.

3. The compound of Claim 2 wherein AA₁₆ is a positively charged amino acid residue selected from Om, homoArg, Lys, *p*-amidinophenyl-Ala and Arg.

4. The compound of Claim 3 wherein AA₁₆ isArg.

5. The compound of Claim 4 wherein X is Thr-Ala-Pro-Arg- (SEQ ID NO: 1).

6. The compound of Claim 2 wherein AA₉ is Arg, Thr or Glu.

7. The compound of Claim 6 wherein AA₁₁ is Asp, Ser or Pro.

8. The compound of Claim 2 wherein AA₁₁ is Asp, Ser or Pro.

9. The compound of Claim 2 having a lower affinity for the human C receptor (hNPR-C) than wild-type hANF(1-28).

10. An ANF variant selected from the group
hANF(1-28) G9T, R11S, M12I;
hANF(1-28) G9R, R11D, M12I, G16R;
hANF(1-28) G9T, R11S;
hANF(1-28) G9E, G10R, R11P;
hANF(1-28) G10K, R11S;
hANF(1-28) M12I, G16R;
hANF(1-28) G9T, R11S, G16R, Q18P;
hANF(TAPR 1-28) M12I, G16R;
hANF(1-28) M12I, R14S, G16R;
hANF(1-28) G9E, G10R, R11P, M12I, G16R;
hANF(1-28) R3D, G9T, R11S, M12L, R14S, G16R;
hANF(1-28) R3D, G9T, R11S, M12L, R14S, G16R, Q18 homocysteine, Y28 homocysteine.

11. A pharmaceutical composition comprising a pharmaceutically acceptable excipient and the compound of any one of claims 1 to 10.

12. A compound of anyone of claims 1 to 10 for use in a method of medical treatment.

13. Use of a compound of anyone of claims I to 10 for the preparation of a medicament for inducing naturesis, diuresis or vasolidation.

## Patentansprüche

1. Verbindung, dargestellt durch die Formel (I) worin
X aus
H, C₁-C₆-Alkanoyl, Ser-Pro-Lys, Thr-Ala-Pro-Arg- (Seq.-ID Nr. 1) und C₁-C₆-Alkanoyl-Thr-Ala-Pro-Arg- (Seq.-ID Nr. 1) ausgewählt ist;
AA₁ fehlt oder ausgewählt ist aus
Ser, Met, Gly, Asp, Ala, Tyr und His;
AA₂ fehlt oder ausgewählt ist aus
Leu, Asp, Met, Val, Ser, Ala, Phe, Pro und Lys;
AA₃ fehlt oder ausgewählt ist aus
Cys, Glu, Ser, Gln, His, Gly, Arg und Asp;
AA₄ fehlt oder ausgewählt ist aus
Arg, Gly, Ala, Asp, Met, Leu, Tyr und Pro;
AA₅ fehlt oder ausgewählt ist aus
Ser, Cys und Asp;
AA₆ fehlt oder ausgewählt ist aus
Ser, Gly und Glu;
AA₇ fehlt oder ausgewählt ist aus
Cys, N-Methyl-Cys, D-Cys und Pen;
AA₈ fehlt oder ausgewählt ist aus
Phe, N-Methyl-Phe, Trimethylphenyl-Ala, Halo-(F-, Cl-, Br- oder I-)phenyl-Ala, Trifluormethylphenyl-Ala, Tyr, O-Methyl-Tyr, Cha, β-Naphthyl-Ala, α-Naphthyl-Ala, Biphenyl-Ala, Diphenyl-Ala, Dibenzyl-Ala, Florenyl-Ala, Adamantyl-Ala und β-Napthyloxy-Ala;
AA₉ ausgewählt ist aus
Gly, Arg, Thr, Val, Asp, Ala, Pro und Glu;
AA₁₀ ausgewählt ist aus
Gly, Arg, Ser, Ala, His, Pro und Lys;
AA₁₁ ausgewählt ist aus
Arg, Lys, N-Methyl-Arg, Asp, Ser und Pro;
AA₁₂ ausgewählt ist aus
Met, Ile, D-Leu, Nle und Leu;
AA₁₃ ausgewählt ist aus
Asp und Glu;
AA₁₄ ausgewählt ist aus
Arg, N-Methyl-Arg, Pro und Ser;
AA₁₅ ausgewählt ist aus
Ile und Leu;
AA₁₆ ausgewählt ist aus
Gly, Tyr, Phe, Ser, Tyr, Pro und einem positiv geladenen Aminosäurerest, ausgewählt aus Orn, Har, Lys, p-Amidinophenyl-Ala und Arg;
AA₁₇ ausgewählt ist aus
Ala, Ser, N-Methyl-Ala und Pro;
AA₁₈ ausgewählt ist aus
Gln, Ser und Homo-Cys;
AA₁₉ ausgewählt ist aus
Ser;
AA₂₀ ausgewählt ist aus
Gly und Ala;
AA₂₁ ausgewählt ist aus
Leu;
AA₂₂ ausgewählt ist aus
Gly und Ala;
AA₂₃ ausgewählt ist aus
Cys;
AA₂₄ ausgewählt ist aus
Asn;
AA₂₅ ausgewählt ist aus
Ser und Val;
AA₂₆ ausgewählt ist aus
Phe und Leu;
AA₂₇ ausgewählt ist aus
Arg, Orn, Har, Lys, p-Amidinophenyl-Ala und Arg-Arg;
AA₂₈ ausgewählt ist aus
Tyr, Arg, Orn, Har, Lys, p-Amidinophenyl-Ala und Homo-Cys; und Z ausgewählt ist aus
OH und NR¹R², worin R¹ und R² unabhängig voneinander ausgewählt sind aus H, C₁-C₆-Alkyl, C₆-C₁₂-Aryl und C₆-C₁₂-Aryl-C₁-C₆-alkyl;
und worin
die Amidbindung (-C(=0)-NH-), welche die Reste AA₇ und AA₈ bindet, gegebenenfalls durch ein Amid-Isoster ersetzt sein kann, ausgewählt aus der Gruppe
-CH₂-NH-,
-CH₂-S-,
-CH₂-S(O)ₙ-, worin n = 1 oder 2 ist
-CH₂-CH₂-,
-CH=CH- (E or Z),
-C(=O)-CH₂-,
-CH(CN)-NH-,
-C(OH)-CH₂-, und
-O-C(=O)-NH-
mit der Maßgabe, dass eine von AA₉, AA₁₁ und AA₁₆ gemäß dem folgenden Schema ausgewählt ist:
AA₉ ist Arg, Thr oder Glu;
AA₁₁ ist Asp, Ser oder Pro; und
AA₁₆ ist ein positiv geladener Aminosäurerest, ausgewählt aus
Arg, Homo-Arg, Lys, Orn und p-Amidinophenyl-Ala;
und pharmazeutisch annehmbare Salze davon, mit der Maßgabe, dass, wenn AA₁₁ Pro ist, AA₉ oder AA₁₆ nicht Gly, Ala oder Pro ist.

2. Verbindung dargestellt durch die Formel (II) (Seq.-ID Nr. 47) 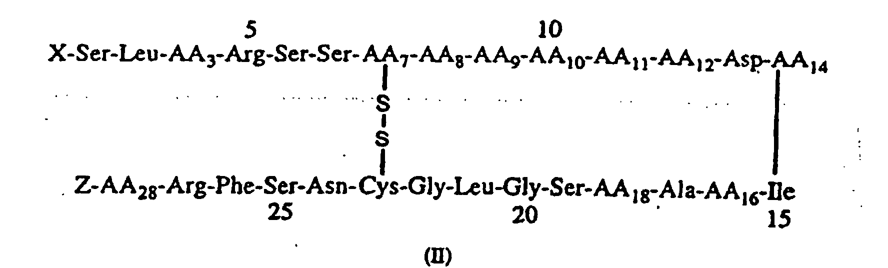 worin
X ausgewählt ist aus
H, C₁-C₆-Alkanoyl und Thr-Ala-Pro-Arg- (Seq.-ID Nr. 1);
AA₃ ausgewählt ist aus
Arg oder Asp;
AA₇ ausgewählt ist aus
Cys, N-Methyl-Cys, D-Cys und Pen;
AA₈ ausgewählt ist aus
Phe, N-Methyl-Phe, Trimethylphenyl-Ala, Fluorphenyl-Ala, Trifluormethylphenyl-Ala, Tyr, O-Methyl-Tyr, Cha, β-Naphthyl-Ala, α-Naphthyl-Ala, Biphenyl-Ala, Diphenyl-Ala, Dibenzyl-Ala, Florenyl-Ala, Adamantyl-Ala, 2-Thienyl-Ala und β-Naphtyloxy-Ala;
AA₉ ausgewählt ist aus
Gly, Arg, Thr, Val, Asp und Glu;
AA₁₀ ausgewählt ist aus
Gly, Arg, Ser, Ala, His und Lys;
AA₁₁ ausgewählt ist aus
Arg, Asp, Ser und Pro;
AA₁₂ ausgewählt ist aus
Met, lle, D-Leu, Leu und Nle;
AA₁₄ ausgewählt ist aus
Arg und Ser;
AA₁₆ ausgewählt ist aus
Gly, Phe, Tyr und einem positiv geladenen Aminosäurerest, ausgewählt aus
Orn, Har, Lys, p-Amidinophenyl-Ala und Arg;
AA₁₈ ausgewählt ist aus
Gln und Homo-Cys;
AA₂₈ ausgewählt ist aus
Tyr und Homo-Cys; und
Z ausgewählt ist aus
OH und NH₂;
und worin
die Amidbindung (-C(=0)-NH-), welche die Reste AA₇ und AA₈ bindet, gegebenenfalls durch ein Amid-Isoster ersetzt sein kann, ausgewählt aus der Gruppe
-CH₂-NH-,
-CH₂-S-,
-CH₂-S(O)ₙ-, worin n = 1 oder 2 ist
-CH₂-CH₂-,
-CH=CH-(E or Z),
-C(=O)-CH₂-,
-CH(CN)-NH-,
-C(OH)-CH₂-, und
-O-C(=O)-NH-
mit der Maßgabe, dass eine von AA₉, AA₁₁ und AA₁₆ gemäß dem folgenden Schema ausgewählt ist:
AA₉ ist Arg, Thr oder Glu;
AA₁₁ ist Asp, Ser oder Pro; und
AA₁₆ ist ein positiv geladener Aminosäurerest, ausgewählt aus
Orn, Har, Lys, p-Amidinophenyl-Ala und Arg;
und pharmazeutisch annehmbare Salze davon, mit der Maßgabe, dass, wenn AA₁₁ Pro ist, AA₉ oder AA₁₆ nicht Gly, Ala oder Pro ist.

3. Verbindung nach Anspruch 2, worin AA₁₆ ein positiv geladener Aminosäurerest, ausgewählt aus Orn, Homo-Arg, Lys, p-Amidinophenyl-Ala und Arg ist.

4. Verbindung nach Anspruch 3, worin AA₁₆ Arg ist.

5. Verbindung nach Anspruch 4, worin X Thr-Ala-Pro-Arg (Seq.-ID Nr. 1) ist.

6. Verbindung nach Anspruch 2, worin AA₉ Arg, Thr oder Glu ist.

7. Verbindung nach Anspruch 6, worin AA₁₁ Asp, Ser oder Pro ist.

8. Verbindung nach Anspruch 2, worin AA₁₁ Asp, Ser oder Pro ist.

9. Verbindung nach Anspruch 2 mit geringerer Affinität für den Human C-Rezeptor (hNPR-C) als hANF(1-28) der Wildform.

10. ANF-Variante, ausgewählt aus der Gruppe:
hANF(1-28) G9T, R11S, M12I;
hANF(1-28) G9R, R11D, M12I, G16R;
hANF(1-28) G9T, R11S;
hANF(1-28) G9E, G10R, R11P;
hANF(1-28) G10K, R11S;
hANF(1-28) M12I, G16R;
hANF(1-28) G9T, R11S, G16R, Q18P;
hANF(TAPR 1-28) M12I, G16R;
hANF(1-28) M12I, R14S, G16R;
hANF(1-28) G9E, G10R, R11P, M12I, G16R;
hANF(1-28) R3D, G9T, R11S, M12L, R14S, G16R;
hANF(1-28) R3D, G9T, R11S, M12L, R14S, G16R, Q18-Homocystein, Y28-Homocystein.

11. Pharmazeutische Zusammensetzung, umfassend einen pharmazeutisch annehmbaren Exzipienten und eine Verbindung nach einem der Ansprüche 1 bis 10.

12. Verbindung nach einem der Ansprüche 1 bis 10 zur Verwendung in einem medizinischen Behandlungsverfahren.

13. Verwendung einer Verbindung nach einem der Ansprüche 1 bis 10 zur Herstellung eines Medikaments, um Naturese, Diurese oder Vasodilatation zu induzieren.

## Revendications

1. Composé représenté par la formule (I) 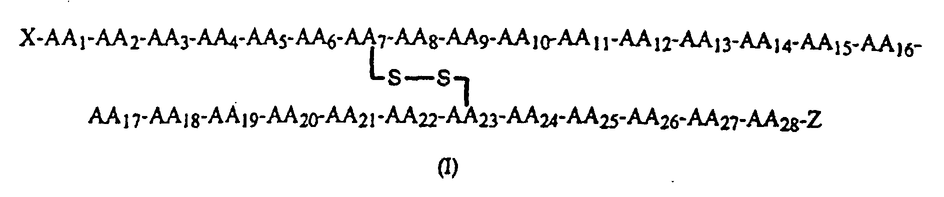 dans laquelle
X est choisi entre des groupes
H, alcanoyle en C₁ à C₆, Ser-Pro-Lys-, Thr-Ala-Pro-Arg- (SEQ ID N° 1) et (alcanoyle en C₁ à C₆)-Thr-Ala-Pro-Arg- (SEQ ID N° 1) ;
AA₁ est absent ou est choisi entre
Ser, Met, Gly, Asp, Ala, Tyr et His ;
AA₂ est absent ou est choisi entre
Leu, Asp, Met, Val, Ser, Ala, Phe, Pro et Lys ;
AA₃ est absent ou est choisi entre
Cys, Glu, Ser, Gln, His, Gly, Arg et Asp ;
AA₄ est absent ou est choisi entre
Arg, Gly, Ala, Asp, Met, Leu, Tyr et Pro ;
AA₅ est absent ou est choisi entre
Ser, Cys et Asp ;
AA₆ est absent ou est choisi entre
Ser, Gly et Glu ;
AA₇ est choisi entre
Cys, N-méthyl-Cys, D-Cys et Pen ;
AA₈ est choisi entre
Phe, N-méthyl-Phe, triméthylphényl-Ala, halogéno-(F, Cl, Br et I)phényl-Ala, trifluorométhylphényl-Ala, Tyr, O-méthyl-Tyr, Cha, β-naphtyl-Ala, α-naphtyl-Ala, biphényl-Ala, diphényl-Ala, dibenzyl-Ala, fluorényl-Ala, adamantyl-Ala et β-naphtyloxy-Ala ;
AA₉ est choisi entre
Gly, Arg, Thr, Val, Asp, Ala, Pro et Glu ;
AA₁₀ est choisi entre
Gly, Arg, Ser, Ala, His, Pro et Lys ;
AA₁₁ est choisi entre
Arg, Lys, N-méthyl-Arg, Asp, Ser et Pro ;
AA₁₂ est choisi entre
Met, Ile, D-Leu, Nle et Leu ;
AA₁₃ est choisi entre
Asp et Glu ;
AA₁₄ est choisi entre
Arg, N-méthyl-Arg, Pro et Ser ;
AA₁₅ est choisi entre
Ile et Leu ;
AA₁₆ est choisi entre
Gly, Tyr, Phe, Ser, Tyr, Pro et un résidu d'aminoacide chargé positivement choisi entre Orn, Har, Lys, p-amidinophényl-Ala et Arg ;
AA₁₇ est choisi entre
Ala, Ser, N-méthyl-Ala et Pro ;
AA₁₈ est choisi entre
Gln, Ser et homo-Cys ;
AA₁₉ représente
Ser ;
AA₂₀ est choisi entre
Gly et Ala ;
AA₂₁ représente
Leu ;
AA₂₂ est choisi entre
Gly et Ala ;
AA₂₃ représente
Cys ;
AA₂₄ représente
Asn ;
AA₂₅ est choisi entre
Ser et Val ;
AA₂₆ est choisi entre
Phe et Leu ;
AA₂₇ est choisi entre
Arg, Orn, Har, Lys, p-amidinophényl-Ala et Arg-Arg ;
AA₂₈ est choisi entre
Tyr, Arg, Orn, Har, Lys, p-amidinophényl-Ala et homoCys ; et
Z est choisi entre
OH et NR¹R² dans lequel R¹ et R² sont choisis indépendamment entre H, des groupes alkyle en C₁ à C₆, aryle en C₆ à C₁₂ et (aryle en C₆ à C₁₂) - (alkyle en C₁ à C₆) ;
et
la liaison amide (-C(=O)-NH- reliant les résidus AA₇ et AA₈ peut être facultativement remplacée par un isostère d'amide choisi dans le groupe consistant en
-CH₂-NH-,
-CH₂-S-,
-CH₂-S(O)ₙ-, dans lequel n est égal à 1 ou 2,
-CH₂-CH₂-,
-CH=CH- (E ou Z),
-C(=O)-CH₂-,
-CH(CN)-NH-,
-C(OH)-CH₂-, et
-O-C (=O) -NH-
sous réserve qu'un des groupes AA₉, AA₁₁ et AA₁₆ soit choisi d'après le schéma suivant
AA₉ représente Arg, Thr ou Glu ;
AA₁₁ représente Asp, Ser ou Pro ; et
AA₁₆ représente un résidu d'aminoacide chargé positivement choisi entre
Arg, homoArg, Lys, Orn, et p-amidinophényl-Ala ; et ses sels pharmaceutiquement acceptables, sous réserve que, lorsque AA₁ représente Pro, AA₉ ou AA₁₆ soit autre que Gly, Ala ou Pro.

2. Composé représenté par la formule (II) (SEQ ID N° 47) 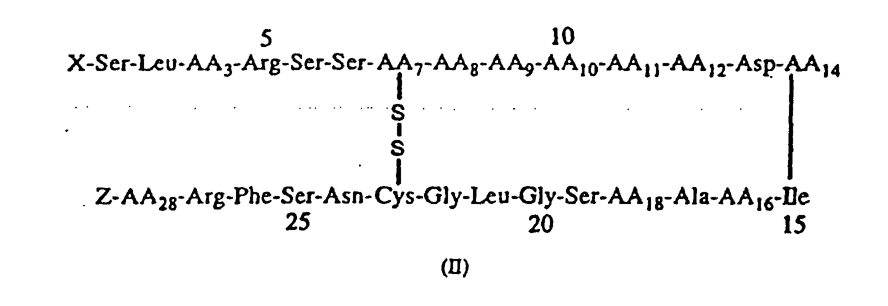 dans laquelle
X est choisi entre
H, alcanoyle en C₁ à C₆ et Thr-Ala-Pro-Arg- (SEQ ID N° 1) ;
AA₃ est choisi entre
Arg et Asp ;
AA₇ est choisi entre
Cys, N-méthyl-Cys, D-Cys et Pen ;
AA₈ est choisi entre
Phe, N-méthyl-Phe, triméthylphényl-Ala, fluorophényl-Ala, trifluorométhylphényl-Ala, Tyr, O-méthyl-Tyr, Cha, β-naphtyl-Ala, α-naphtyl-Ala, biphényl-Ala, diphényl-Ala-, dibenzyl-Ala, florényl-Ala, adamantyl-Ala, 2-thiényl-Ala et β-naphtyloxy-Ala ;
AA₉ est choisi entre
Gly, Arg, Thr, Val, Asp et Glu ;
AA₁₀ est choisi entre
Gly, Arg, Ser, Ala, His et Lys ;
AA₁₁ est choisi entre
Arg, Asp, Ser et Pro ;
AA₁₂ est choisi entre
Met, Ile, D-Leu, Leu et Nle ;
AA₁₄ est choisi entre
Arg et Ser ;
AA₁₆ est choisi entre
Gly, Phe, Tyr et un résidu d'aminoacide chargé positivement choisi entre
Orn, Har, Lys, p-amidinophényl-Ala et Arg ;
AA₁₈ est choisi entre
Gln et homoCys ;
AA₂₈ est choisi entre
Tyr et homoCys ; et
Z est choisi entre
OH et NH₂ ;
et
la liaison amide (-C(=O)-NH-) reliant les résidus AA₇ et AA₈ peut être facultativement remplacée par un isostère d'amide choisi dans le groupe consistant en
-CH₂-NH-,
-CH₂-S-,
-CH₂-S(O)ₙ-, dans lequel n est égal à 1 ou 2,
-CH₂-CH₂-,
-CH=CH- (E ou Z),
-C(=O)-CH₂-,
-CH(CN)-NH-,
-C(OH)-CH₂-, et
-O-C(=O)-NH-
sous réserve qu'un des groupes AA₉, AA₁₁ et AA₁₆ soit choisi d'après le schéma suivant
AA₉ représente Arg, Thr ou Glu ;
AA₁₁ représente Asp, Ser ou Pro ; et
AA₁₆ représente un résidu d'aminoacide chargé positivement choisi entre
Orn, Har, Lys, p-amidinophényl-Ala et Arg ;
et
ses sels pharmaceutiquement acceptables, sous réserve que, lorsque AA₁₁ représente Pro, AA₉ ou AA₁₆ soit autre que Gly, Ala ou Pro.

3. Composé suivant la revendication 2, dans lequel AA₁₆ représente un résidu d'aminoacide chargé positivement choisi entre Orn, homoArg, Lys, p-amidinophényl-Ala et Arg.

4. Composé suivant la revendication 3, dans lequel AA₁₆ représente Arg.

5. Composé suivant la revendication 4, dans lequel X représente Thr-Ala-Pro-Arg (SEQ ID N° 1).

6. Composé suivant la revendication 2, dans lequel AA₉ représente Arg, Thr ou Glu.

7. Composé suivant la revendication 6, dans lequel AA₁₁ représente Asp, Ser ou Pro.

8. Composé suivant la revendication 2, dans lequel AA₁₁ représente Asp, Ser ou Pro.

9. Composé suivant la revendication 2, ayant une plus faible affinité pour le récepteur C humain (hNPR-C) que le hANF (1-28) de type sauvage.

10. Variant de ANF choisi dans le groupe consistant en
hANF(1-28) G9T, R11S, M12I;
hANF(1-28) G9R, R11D, M12I, G16R;
hANF(1-28) G9T, R11S;
hANF(1-28) G9E, G10R, R11P;
hANF(1-28) G10K, R11S;
hANF(1-28) M12I, G16R;
hANF(1-28) G9T, R11S, G16R, Q18P;
hANF(TAPR 1-28) M12I, G16R;
hANF(1-28) M12I, R14S, G16R;
hANF(1-28) G9E, G10R, R11P, M12I, G16R;
hANF(1-28) R3D, G9T, R11S, M12L, R14S, G16R;
hANF(1-28)R3D, G9T, R11S, M12L, R14S, G16R, Q18homocystéine, Y28homocystéine.

11. Composition pharmaceutique comprenant un excipent pharmaceutiquement acceptable et le composé suivant l'une quelconque des revendications 1 à 10.

12. Composé suivant l'une quelconque des revendications 1 à 10, destiné à être utilisé dans une méthode de traitement médical.

13. Utilisation d'un composé suivant l'une quelconque des revendications 1 à 10 pour la préparation d'un médicament destiné à induire une natriurie, une diurèse ou une vasodilatation.


### SEQUENCE LISTING

(1) GENERAL INFORMATION:
   (i) APPLICANT: Genentech, Inc.
      Lowe, David
      Cunningham, Brian C.
      Oare, David
      McDowell, Robert S.
      Burnier, John
   (ii) TITLE OF INVENTION: RECEPTOR SPECIFIC ATRIAL NATRIURETIC PEPTIDES
   (iii) NUMBER OF SEQUENCES: 47
   (iv) CORRESPONDENCE ADDRESS:
      (A) ADDRESSEE: Genentech, Inc.
      (B) STREET: 460 Point San Bruno Blvd
      (C) CITY: South San Francisco
      (D) STATE: California
      (E) COUNTRY: USA
      (F) ZIP: 94080
   (v) COMPUTER READABLE FORM:
      (A) MEDIUM TYPE: 5.25 inch, 360 Kb floppy disk
      (B) COMPUTER: IBM PC compatible
      (C) OPERATING SYSTEM: PC-DOS/MS-DOS
      (D) SOFTWARE: patin (Genentech)
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: 08/152994
      (B) FILING DATE: 12-NOV-1993
   (viii) ATTORNEY/AGENT INFORMATION:
      (A) NAME: Winter, Daryl B.
      (B) REGISTRATION NUMBER: 32,637
      (C) REFERENCE/DOCKET NUMBER: 844P1
   (ix) TELECOMMUNICATION INFORMATION:
      (A) TELEPHONE: 415/225-1249
      (B) TELEFAX: 415/952-9881
      (C) TELEX: 910/371-7168
(2) INFORMATION FOR SEQ ID NO:1:
   (i) SEQUENCE CHARACTERISTICS:
      (A) LENGTH: 4 amino acids
      (B) TYPE: amino acid
      (D) TOPOLOGY: linear
   (xi) SEQUENCE DESCRIPTION: SEQ ID NO:1:
   Thr Ala Pro Arg
     1           4
(2) INFORMATION FOR SEQ ID NO:2:
   (i) SEQUENCE CHARACTERISTICS:
      (A) LENGTH: 32 amino acids
      (B) TYPE: amino acid
      (D) TOPOLOGY: linear
   (xi) SEQUENCE DESCRIPTION: SEQ ID NO:2:
   Thr Ala Pro Arg Ser Leu Arg Arg Ser Ser Cys Phe Gly Gly Arg
    1               5                  10                  15
   Met Asp Arg Ile Gly Ala Gln Ser Gly Leu Gly Cys Asn Ser Phe
                   20                  25                  30
   Arg Tyr
       32
(2) INFORMATION FOR SEQ ID NO:3:
   (i) SEQUENCE CHARACTERISTICS:
      (A) LENGTH: 32 amino acids
      (B) TYPE: amino acid
      (D) TOPOLOGY: linear
   (xi) SEQUENCE DESCRIPTION: SEQ ID NO:3:
   Ser Pro Lys Met Val Gln Gly Ser Gly Cys Phe Gly Arg Lys Met
    1               5                  10                  15
   Asp Arg Ile Ser Ser Ser Ser Gly Leu Gly Cys Asn Val Leu Arg
                   20                  25                  30
   Arg Tyr
       32
(2) INFORMATION FOR SEQ ID NO:4:
   (i) SEQUENCE CHARACTERISTICS:
      (A) LENGTH: 22 amino acids
      (B) TYPE: amino acid
      (D) TOPOLOGY: linear
   (xi) SEQUENCE DESCRIPTION: SEQ ID NO:4:
   Gly Leu Ser Lys Gly Cys Phe Gly Leu Lys Leu Asp Arg Ile Gly
    1               5                  10                  15
   Ser Met Ser Gly Leu Gly Cys
                   20      22
(2) INFORMATION FOR SEQ ID NO:5:
   (i) SEQUENCE CHARACTERISTICS:
      (A) LENGTH: 32 amino acids
      (B) TYPE: amino acid
      (D) TOPOLOGY: linear
   (xi) SEQUENCE DESCRIPTION: SEQ ID NO:5:
   Thr Ala Pro Arg Ser Leu Arg Arg Ser Ser Cys Phe Gly Gly Arg
    1               5                  10                  15
   Ile Asp Arg Ile Arg Ala Gln Ser Gly Leu Gly Cys Asn Ser Phe
                   20                  25                  30
   Arg Tyr
       32
(2) INFORMATION FOR SEQ ID NO:6:
   (i) SEQUENCE CHARACTERISTICS:
      (A) LENGTH: 28 amino acids
      (B) TYPE: amino acid
      (D) TOPOLOGY: linear
   (xi) SEQUENCE DESCRIPTION: SEQ ID NO:6:
   Ser Leu Arg Arg Ser Ser Cys Phe Gly Gly Arg Ile Asp Arg Ile
    1               5                  10                  15
   Arg Ala Gln Ser Gly Leu Gly Cys Asn Ser Phe Arg Tyr
                   20                  25          28
(2) INFORMATION FOR SEQ ID NO:7:
   (i) SEQUENCE CHARACTERISTICS:
      (A) LENGTH: 15 amino acids
      (B) TYPE: amino acid
      (D) TOPOLOGY: linear
   (xi) SEQUENCE DESCRIPTION: SEQ ID NO:7:
   Ser Ser Cys Xaa Phe Gly Gly Arg Ile Asp Arg Ile Cys Phe Arg
    1               5                  10                  15
(2) INFORMATION FOR SEQ ID NO:8:
   (i) SEQUENCE CHARACTERISTICS:
      (A) LENGTH: 28 amino acids
      (B) TYPE: amino acid
      (D) TOPOLOGY: linear
   (xi) SEQUENCE DESCRIPTION: SEQ ID NO:8:
   Ser Leu Arg Arg Ser Ser Cys Phe Thr Gly Ser Met Asp Arg Ile
    1               5                  10                  15
   Gly Ala Gln Ser Gly Leu Gly Cys Asn Ser Phe Arg Tyr
                   20                  25          28
(2) INFORMATION FOR SEQ ID NO:9:
   (i) SEQUENCE CHARACTERISTICS:
      (A) LENGTH: 28 amino acids
      (B) TYPE: amino acid
      (D) TOPOLOGY: linear
   (xi) SEQUENCE DESCRIPTION: SEQ ID NO:9:
   Ser Leu Arg Arg Ser Ser Cys Phe Gly Gly Arg Ile Asp Arg Ile
     1             5                   10                  15
   Gly Ala Gln Ser Gly Leu Gly Cys Asn Ser Phe Arg Tyr
                   20                  25          28
(2) INFORMATION FOR SEQ ID NO:10:
   (i) SEQUENCE CHARACTERISTICS:
      (A) LENGTH: 28 amino acids
      (B) TYPE: amino acid
      (D) TOPOLOGY: linear
   (xi) SEQUENCE DESCRIPTION: SEQ ID NO:10:
   Ser Leu Arg Arg Ser Ser Cys Phe Gly Gly Arg Ile Asp Ser Ile
     1             5                   10                  15
   Arg Ala Gln Ser Gly Leu Gly Cys Asn Ser Phe Arg Tyr
                   20                  25          28
(2) INFORMATION FOR SEQ ID NO:11:
   (i) SEQUENCE CHARACTERISTICS:
      (A) LENGTH: 28 amino acids
      (B) TYPE: amino acid
      (D) TOPOLOGY: linear
   (xi) SEQUENCE DESCRIPTION: SEQ ID NO:11:
   Ser Leu Arg Arg Ser Ser Cys Phe Thr Gly Ser Ile Asp Arg Ile
     1             5                   10                  15
   Arg Ala Gln Ser Gly Leu Gly Cys Asn Ser Phe Arg Tyr
                   20                  25          28
(2) INFORMATION FOR SEQ ID NO:12:
   (i) SEQUENCE CHARACTERISTICS:
      (A) LENGTH: 28 amino acids
      (B) TYPE: amino acid
      (D) TOPOLOGY: linear
   (xi) SEQUENCE DESCRIPTION: SEQ ID NO:12:
   Ser Leu Arg Arg Ser Ser Cys Phe Arg Gly Asp Ile Asp Arg Ile
    1               5                  10                  15
   Arg Ala Gln Ser Gly Leu Gly Cys Asn Ser Phe Arg Tyr
                   20                  25          28
(2) INFORMATION FOR SEQ ID NO:13:
   (i) SEQUENCE CHARACTERISTICS:
      (A) LENGTH: 28 amino acids
      (B) TYPE: amino acid
      (D) TOPOLOGY: linear
   (xi) SEQUENCE DESCRIPTION: SEQ ID NO:13:
   Ser Leu Arg Arg Ser Ser Cys Phe Thr Gly Ser Ile Asp Arg Ile
    1               5                  10                  15
   Arg Ala Pro Ser Gly Leu Gly Cys Asn Ser Phe Arg Tyr
                   20                  25          28
(2) INFORMATION FOR SEQ ID NO:14:
   (i) SEQUENCE CHARACTERISTICS:
      (A) LENGTH: 28 amino acids
      (B) TYPE: amino acid
      (D) TOPOLOGY: linear
   (xi) SEQUENCE DESCRIPTION: SEQ ID NO:14:
   Ser Leu Arg Arg Ser Ser Cys Phe Gly Lys Ser Met Asp Arg Ile
    1               5                  10                  15
   Gly Ala Gln Ser Gly Leu Gly Cys Asn Ser Phe Arg Tyr
                   20                  25          28
(2) INFORMATION FOR SEQ ID NO:15:
   (i) SEQUENCE CHARACTERISTICS:
      (A) LENGTH: 32 amino acids
      (B) TYPE: amino acid
      (D) TOPOLOGY: linear
   (xi) SEQUENCE DESCRIPTION: SEQ ID NO:15:
   Thr Ala Pro Arg Ser Leu Arg Arg Ser Ser Cys Phe Thr Gly Ser
     1               5                  10                  15
   Ile Asp Arg Ile Arg Ala Gln Ser Gly Leu Gly Cys Asn Ser Phe
                    20                  25                  30
   Arg Tyr
        32
(2) INFORMATION FOR SEQ ID NO:16:
   (i) SEQUENCE CHARACTERISTICS:
      (A) LENGTH: 28 amino acids
      (B) TYPE: amino acid
      (D) TOPOLOGY: linear
   (xi) SEQUENCE DESCRIPTION: SEQ ID NO:16:
   Ser Leu Arg Arg Ser Ser Cys Xaa Thr Gly Ser Ile Asp Arg Ile
     1               5                  10                  15
   Arg Ala Gln Ser Gly Leu Gly Cys Asn Ser Phe Arg Tyr
                    20                  25          28
(2) INFORMATION FOR SEQ ID NO:17:
   (i) SEQUENCE CHARACTERISTICS:
      (A) LENGTH: 28 amino acids
      (B) TYPE: amino acid
      (D) TOPOLOGY: linear
   (xi) SEQUENCE DESCRIPTION: SEQ ID NO:17:
   Ser Leu Arg Arg Ser Ser Xaa Phe Thr Gly Ser Ile Asp Arg Ile
     1               5                  10                  15
   Arg Ala Gln Ser Gly Leu Gly Cys Asn Ser Phe Arg Tyr
                    20                  25          28
(2) INFORMATION FOR SEQ ID NO:18:
   (i) SEQUENCE CHARACTERISTICS:
      (A) LENGTH: 29 amino acids
      (B) TYPE: amino acid
      (D) TOPOLOGY: linear
   (xi) SEQUENCE DESCRIPTION: SEQ ID NO:18:
   Ser Leu Arg Arg Ser Ser Cys Asn Xaa Thr Gly Ser Ile Asp Arg
    1               5                  10                  15
   Ile Arg Ala Gln Ser Gly Leu Gly Cys Asn Ser Phe Arg Tyr
                   20                  25              29
(2) INFORMATION FOR SEQ ID NO:19:
   (i) SEQUENCE CHARACTERISTICS:
      (A) LENGTH: 27 amino acids
      (B) TYPE: amino acid
      (D) TOPOLOGY: linear
   (xi) SEQUENCE DESCRIPTION: SEQ ID NO:19:
   Ser Leu Arg Arg Ser Ser Xaa Thr Gly Ser Ile Asp Arg Ile Arg
    1               5                  10                  15
   Ala Gln Ser Gly Leu Gly Cys Asn Ser Phe Arg Tyr
                   20                  25      27
(2) INFORMATION FOR SEQ ID NO:20:
   (i) SEQUENCE CHARACTERISTICS:
      (A) LENGTH: 22 amino acids
      (B) TYPE: amino acid
      (D) TOPOLOGY: linear
   (xi) SEQUENCE DESCRIPTION: SEQ ID NO:20:
   Xaa Phe Xaa Gly Asp Ile Asp Arg Ile Arg Ala Gln Ser Gly Leu
    1               5                  10                  15
   Gly Cys Asn Ser Phe Arg Tyr
                   20      22
(2) INFORMATION FOR SEQ ID NO:21:
   (i) SEQUENCE CHARACTERISTICS:
      (A) LENGTH: 28 amino acids
      (B) TYPE: amino acid
      (D) TOPOLOGY: linear
   (xi) SEQUENCE DESCRIPTION: SEQ ID NO:21:
   Ser Leu Arg Arg Ser Ser Cys Phe Gly Gly Arg Met Asp Arg Ile
    1              5                   10                  15
   Gly Ala Gln Ser Gly Leu Gly Cys Asn Ser Phe Arg Tyr
                   20                  25          28
(2) INFORMATION FOR SEQ ID NO:22:
   (i) SEQUENCE CHARACTERISTICS:
      (A) LENGTH: 32 amino acids
      (B) TYPE: amino acid
      (D) TOPOLOGY: linear
   (xi) SEQUENCE DESCRIPTION: SEQ ID NO:22:
   Ser Pro Lys Met Val Gln Gly Ser Gly Cys Phe Gly Arg Lys Met
    1              5                   10                  15
   Asp Arg Ile Ser Ser Ser Ser Gly Leu Gly Cys Lys Val Leu Arg
                   20                  25                  30
   Arg His
       32
(2) INFORMATION FOR SEQ ID NO:23:
   (i) SEQUENCE CHARACTERISTICS:
      (A) LENGTH: 28 amino acids
      (B) TYPE: amino acid
      (D) TOPOLOGY: linear
   (xi) SEQUENCE DESCRIPTION: SEQ ID NO:23:
   Ser Leu Arg Arg Ser Ser Cys Phe Thr Gly Ser Met Asp Arg Ile
    1              5                   10                  15
   Arg Ala Gln Ser Gly Leu Gly Cys Asn Ser Phe Arg Tyr
                   20                  25          28
(2) INFORMATION FOR SEQ ID NO:24:
   (i) SEQUENCE CHARACTERISTICS:
      (A) LENGTH: 28 amino acids
      (B) TYPE: amino acid
      (D) TOPOLOGY: linear
   (xi) SEQUENCE DESCRIPTION: SEQ ID NO:24:
   Ser Leu Arg Arg Ser Ser Cys Phe Thr Gly Ser Met Asp Arg Ile
    1              5                   10                  15
   Arg Ala Pro Ser Gly Leu Gly Cys Asn Ser Phe Arg Tyr
                   20                  25          28
(2) INFORMATION FOR SEQ ID NO:25:
   (i) SEQUENCE CHARACTERISTICS:
      (A) LENGTH: 28 amino acids
      (B) TYPE: amino acid
      (D) TOPOLOGY: linear
   (xi) SEQUENCE DESCRIPTION: SEQ ID NO:25:
   Ser Leu Arg Arg Ser Ser Cys Phe Glu Arg Pro Met Asp Arg Ile
    1              5                   10                  15
   Gly Ala Gln Ser Gly Leu Gly Cys Asn Ser Phe Arg Tyr
                   20                  25          28
(2) INFORMATION FOR SEQ ID NO:26:
   (i) SEQUENCE CHARACTERISTICS:
      (A) LENGTH: 28 amino acids
      (B) TYPE: amino acid
      (D) TOPOLOGY: linear
   (xi) SEQUENCE DESCRIPTION: SEQ ID NO:26:
   Ser Leu Asp Arg Ser Ser Cys Phe Thr Gly Ser Leu Asp Ser Ile
    1              5                   10                  15
   Arg Ala Gln Ser Gly Leu Gly Cys Asn Ser Phe Arg Tyr
                   20                  25          28
(2) INFORMATION FOR SEQ ID NO:27:
   (i) SEQUENCE CHARACTERISTICS:
      (A) LENGTH: 29 amino acids
      (B) TYPE: amino acid
      (D) TOPOLOGY: linear
   (xi) SEQUENCE DESCRIPTION: SEQ ID NO:27:
   Ser Leu Arg Arg Ser Ser Cys Xaa Phe Thr Gly Ser Ile Asp Arg
     1               5                  10                  15
   Ile Arg Ala Gln Ser Gly Leu Gly Cys Asn Ser Phe Arg Tyr
                    20                  25              29
(2) INFORMATION FOR SEQ ID NO:28:
   (i) SEQUENCE CHARACTERISTICS:
      (A) LENGTH: 29 amino acids
      (B) TYPE: amino acid
      (D) TOPOLOGY: linear
   (xi) SEQUENCE DESCRIPTION: SEQ ID NO:28:
   Ser Leu Arg Arg Ser Ser Cys Xaa Phe Thr Gly Ser Ile Asp Arg
     1               5                  10                  15
   Ile Arg Ala Gln Ser Gly Leu Gly Cys Asn Ser Phe Arg Tyr
                    20                  25              29
(2) INFORMATION FOR SEQ ID NO:29:
   (i) SEQUENCE CHARACTERISTICS:
      (A) LENGTH: 22 amino acids
      (B) TYPE: amino acid
      (D) TOPOLOGY: linear
   (xi) SEQUENCE DESCRIPTION: SEQ ID NO:29:
   Xaa Phe Xaa Gly Ser Ile Asp Arg Ile Arg Ala Gln Ser Gly Leu
     1               5                  10                  15
   Gly Cys Asn Ser Phe Arg Tyr
                    20      22
(2) INFORMATION FOR SEQ ID NO:30:
   (i) SEQUENCE CHARACTERISTICS:
      (A) LENGTH: 22 amino acids
      (B) TYPE: amino acid
      (D) TOPOLOGY: linear
   (xi) SEQUENCE DESCRIPTION: SEQ ID NO:30:
   Xaa Phe Xaa Gly Ser Ile Asp Arg Ile Arg Ala Gln Ser Gly Leu
     1               5                  10                  15
   Gly Cys Asn Ser Phe Arg Tyr
                   20      22
(2) INFORMATION FOR SEQ ID NO:31:
   (i) SEQUENCE CHARACTERISTICS:
      (A) LENGTH: 28 amino acids
      (B) TYPE: amino acid
      (D) TOPOLOGY: linear
   (xi) SEQUENCE DESCRIPTION: SEQ ID NO:31:
   Ser Leu Arg Arg Ser Ser Xaa Phe Thr Gly Ser Ile Asp Arg Ile
    1               5                  10                  15
   Arg Ala Gln Ser Gly Leu Gly Cys Asn Ser Phe Arg Tyr
                   20                  25          28
(2) INFORMATION FOR SEQ ID NO:32:
   (i) SEQUENCE CHARACTERISTICS:
      (A) LENGTH: 28 amino acids
      (B) TYPE: amino acid
      (D) TOPOLOGY: linear
   (xi) SEQUENCE DESCRIPTION: SEQ ID NO:32:
   Ser Leu Arg Arg Ser Ser Cys Xaa Thr Gly Ser Ile Asp Arg Ile
    1               5                  10                  15
   Arg Ala Gln Ser Gly Leu Gly Cys Asn Ser Phe Arg Tyr
                   20                  25          28
(2) INFORMATION FOR SEQ ID NO:33:
   (i) SEQUENCE CHARACTERISTICS:
      (A) LENGTH: 27 amino acids
      (B) TYPE: amino acid
      (D) TOPOLOGY: linear
   (xi) SEQUENCE DESCRIPTION: SEQ ID NO:33:
   Ser Leu Arg Arg Ser Ser Xaa Thr Gly Ser Ile Asp Arg Ile Arg
    1               5                  10                  15
   Ala Gln Ser Gly Leu Gly Cys Asn Ser Phe Arg Tyr
                   20                  25      27
(2) INFORMATION FOR SEQ ID NO:34:
   (i) SEQUENCE CHARACTERISTICS:
      (A) LENGTH: 28 amino acids
      (B) TYPE: amino acid
      (D) TOPOLOGY: linear
   (xi) SEQUENCE DESCRIPTION: SEQ ID NO:34:
   Ser Leu Arg Arg Ser Ser Cys Phe Xaa Gly Asp Ile Asp Arg Ile
     1               5                  10                  15
   Arg Ala Gln Ser Gly Leu Gly Cys Asn Ser Phe Arg Tyr
                    20                  25          28
(2) INFORMATION FOR SEQ ID NO:35:
   (i) SEQUENCE CHARACTERISTICS:
      (A) LENGTH: 28 amino acids
      (B) TYPE: amino acid
      (D) TOPOLOGY: linear
   (xi) SEQUENCE DESCRIPTION: SEQ ID NO:35:
   Ser Leu Arg Arg Ser Ser Cys Xaa Thr Gly Ser Ile Asp Arg Ile
     1               5                  10                  15
   Arg Ala Gln Ser Gly Leu Gly Cys Asn Ser Phe Arg Tyr
                    20                  25          28
(2) INFORMATION FOR SEQ ID NO:36:
   (i) SEQUENCE CHARACTERISTICS:
      (A) LENGTH: 28 amino acids
      (B) TYPE: amino acid
      (D) TOPOLOGY: linear
   (xi) SEQUENCE DESCRIPTION: SEQ ID NO:36:
   Ser Leu Asp Arg Ser Ser Cys Phe Thr Gly Ser Leu Asp Ser Ile
     1               5                  10                  15
   Arg Ala Xaa Ser Gly Leu Gly Cys Asn Ser Phe Arg Xaa
                    20                  25          28
(2) INFORMATION FOR SEQ ID NO:37:
   (i) SEQUENCE CHARACTERISTICS:
      (A) LENGTH: 28 amino acids
      (B) TYPE: amino acid
      (D) TOPOLOGY: linear
   (xi) SEQUENCE DESCRIPTION: SEQ ID NO:37:
   Ser Leu Asp Arg Ser Ser Cys Phe Thr Gly Ser Leu Asp Ser Ile
     1               5                  10                  15
   Arg Ala Xaa Ser Gly Leu Gly Cys Asn Ser Phe Arg Xaa
                    20                  25          28
(2) INFORMATION FOR SEQ ID NO:38:
   (i) SEQUENCE CHARACTERISTICS:
      (A) LENGTH: 36 bases
      (B) TYPE: nucleic acid
      (C) STRANDEDNESS: single
      (D) TOPOLOGY: linear
   (xi) SEQUENCE DESCRIPTION: SEQ ID NO:38:
   TCTCTGCGTA GATCTAGCTG CTTCGGCGGC CGCATG 36
(2) INFORMATION FOR SEQ ID NO:39:
   (i) SEQUENCE CHARACTERISTICS:
      (A) LENGTH: 35 bases
      (B) TYPE: nucleic acid
      (C) STRANDEDNESS: single
      (D) TOPOLOGY: linear
   (xi) SEQUENCE DESCRIPTION: SEQ ID NO:39:
   GATCGTATCG GAGCTCAGAG CGGTCTCGGG TGCAA 35
(2) INFORMATION FOR SEQ ID NO:40:
   (i) SEQUENCE CHARACTERISTICS:
      (A) LENGTH: 24 bases
      (B) TYPE: nucleic acid
      (C) STRANDEDNESS: single
      (D) TOPOLOGY: linear
   (xi) SEQUENCE DESCRIPTION: SEQ ID NO:40:
   CAGCTTCCGT TACGTAGGCG GGCC 24
(2) INFORMATION FOR SEQ ID NO:41:
   (i) SEQUENCE CHARACTERISTICS:
      (A) LENGTH: 24 bases
      (B) TYPE: nucleic acid
      (C) STRANDEDNESS: single
      (D) TOPOLOGY: linear
   (xi) SEQUENCE DESCRIPTION: SEQ ID NO:41:
   CAGCTAGATC TACGCAGAGA TGCA 24
(2) INFORMATION FOR SEQ ID NO:42:
   (i) SEQUENCE CHARACTERISTICS:
      (A) LENGTH: 35 bases
      (B) TYPE: nucleic acid
      (C) STRANDEDNESS: single
      (D) TOPOLOGY: linear
   (xi) SEQUENCE DESCRIPTION: SEQ ID NO:42:
   TCTGAGCTCC GATACGATCC ATGCGGCCGC CGAAG 35
(2) INFORMATION FOR SEQ ID NO:43:
   (i) SEQUENCE CHARACTERISTICS:
      (A) LENGTH: 36 bases
      (B) TYPE: nucleic acid
      (C) STRANDEDNESS: single
      (D) TOPOLOGY: linear
   (xi) SEQUENCE DESCRIPTION: SEQ ID NO:43:
   CGCCTACGTA ACGGAAGCTG TTGCACCCGA GACCGC 36
(2) INFORMATION FOR SEQ ID NO:44:
   (i) SEQUENCE CHARACTERISTICS:
      (A) LENGTH: 27 bases
      (B) TYPE: nucleic acid
      (C) STRANDEDNESS: single
      (D) TOPOLOGY: linear
   (xi) SEQUENCE DESCRIPTION: SEQ ID NO:44:
   AACAGCTTCC GTTACTAGGG CGGGCCC 27
(2) INFORMATION FOR SEQ ID NO:45:
   (i) SEQUENCE CHARACTERISTICS:
      (A) LENGTH: 30 bases
      (B) TYPE: nucleic acid
      (C) STRANDEDNESS: single
      (D) TOPOLOGY: linear
   (xi) SEQUENCE DESCRIPTION: SEQ ID NO:45:
   GCCTATGCAT CTCTGGATAG ATCTAGCTGC 30
(2) INFORMATION FOR SEQ ID NO:46:
   (i) SEQUENCE CHARACTERISTICS:
      (A) LENGTH: 50 bases
      (B) TYPE: nucleic acid
      (C) STRANDEDNESS: single
      (D) TOPOLOGY: linear
   (xi) SEQUENCE DESCRIPTION: SEQ ID NO:46:
   AGATCTAGCT GCTTCACCGG CAGCCTGGAT AGTATCAGAG CTCAGAGCGG 50
(2) INFORMATION FOR SEQ ID NO:47:
   (i) SEQUENCE CHARACTERISTICS:
      (A) LENGTH: 30 amino acids
      (B) TYPE: amino acid
      (D) TOPOLOGY: linear
   (xi) SEQUENCE DESCRIPTION: SEQ ID NO:47:
   Xaa Ser Leu Xaa Arg Ser Ser Xaa Xaa Xaa Xaa Xaa Xaa Asp Xaa
    1               5                  10                  15
   Ile Xaa Ala Xaa Ser Gly Leu Gly Cys Asn Ser Phe Arg Xaa Xaa
                   20                  25                  30